# EUROPÄISCHE PATENTSCHRIFT

(11) **EP 1 875 905 B1**
(45) Veröffentlichungstag und Bekanntmachung des Hinweises auf die Patenterteilung: **12.05.2010**
(21) Anmeldenummer: 07075742.2
(22) Anmeldetag: 21.04.2004
(51) Int. Cl.: A61K 31/265, A61K 31/40, A61K 31/565, A61K 45/06, A61K 47/10, A61K 47/14, A61K 47/24, A61K 47/38, A61K 9/00

(54) **Pharmazeutische Zusammensetzung in Form eines Hydrogels zur transdermalen Verabreichung von Wirkstoffen**
Pharmaceutical compound in the form of a hydrogel for transdermal application of active substances
Composition pharmaceutique sous la forme d'un hydrogel pour l'administration transdermique d'agents actifs

(30) Priorität: 28.04.2003 EP 03008856
(43) Veröffentlichungstag der Anmeldung: 09.01.2008
(62) Teilanmeldung aus: 04728563.0
(73) Patentinhaber: Bayer Schering Pharma Aktiengesellschaft, 13353 Berlin (DE)
(72) Erfinder: Franke, Patrick, 14167 Berlin (DE)

(56) Entgegenhaltungen:
- EP-A- 0 573 133
- EP-A- 0 811 381
- WO-A-88/09185
- WO-A-93/25168
- FR-A- 2 518 879
- PEPPAS N A ET AL: "Hydrogels in pharmaceutical formulations" EUROPEAN JOURNAL OF PHARMACEUTICS AND BIOPHARMACEUTICS, ELSEVIER SCIENCE PUBLISHERS B.V., AMSTERDAM, NL, Bd. 50, Nr. 1, 3. Juli 2000 (2000-07-03), Seiten 27-46, XP004257178 ISSN: 0939-6411

## Beschreibung

Die Erfindung betrifft die Verwendung eines Hydrogels, enthaltend Wirkstoffe (Arzneistoffe), insbesondere Steroidhormone und deren Derivate zur Herstellung eines transdermalen Kontrazeptivums. Eine bevorzugte Ausführungsform betrifft die Verwendung eines Hydrogels, das Propylencarbonat, Ethanol und 7α-Methyl-11β-fluor-19-nortestosteron (eF-MENT) enthält, zur Herstellung eines transdermalen Kontrazeptivums. Die Erfindung betrifft ebenfalls die Verwendung einer pharmazeutischen Zusammensetzung in Form eines Hydrogels, zur Herstellung eines transdermalen Kontrazeptivums, welche besondere physikalische Eigenschaften aufweist, insbesondere erhöhte Schweißfestigkeit, und daher besonders gut auf die Haut aufgetragen werden kann.

Die systemische Verabreichung von Steroidhormonen kann grundsätzlich auf oralem Wege mit Hilfe geeigneter Arzneiformen erfolgen. Häufig ist jedoch die Bioverfügbarkeit bei oraler Verabreichung herabgesetzt, da die Wirkstoffe in der Leber metabolisiert werden, ehe sie in den systemischen Blutkreislauf gelangen (sog. "first-pass-Metabolismus"). Ferner sind in der Regel an die Arzneiformen bei oraler Verabreichung besondere Anforderungen gestellt, da die Abgabe der Steroidhormone möglichst gleichmäßig über einen längeren Zeitraum erfolgen sollte. Um die orale Verabreichung der Steroidhormone zu umgehen, wurden bestimmte Steroid-Derivate entwickelt, welche sich insbesondere zur Injektion eignen. Diesbezüglich kann beispielsweise auf die Druckschriften WO 99/67270 und WO 99/67271 verwiesen werden. Eine andere Möglichkeit, die Steroidhormone kontinuierlich und unter Umgehung des first-pass-Effektes zu verabreichen, besteht in der Verwendung von geeigneten Implantaten. Implantate zur Verabreichung von Androgenen und deren Derivaten werden beispielsweise in folgenden Druckschriften offenbart: EP-A 970 704, WO 97/30656, WO 99/13883, WO 00/28967, US 5,733,565, K. Sundaram et al., Annuals of Medicine, 1993, 25 (2), 199*;* R. A. Anderson et al., J. Clin. Endocrin. & Metab., 1999, 84(10) 3556 und J. Suvisaari et al., Contraception, 1999, 60(5), 299. Diese Implantate haben jedoch den Nachteil, dass sie beim Patienten durch einen operativen Eingriff durch einen Arzt eingesetzt und wieder entnommen werden müssen. Derartige operative Eingriffe bringen stets ein gewisses Infektionsrisiko mit sich.

Ferner besteht in der Bevölkerung eine grundsätzliche Abneigung gegenüber derartigen Darreichungsmethoden, insbesondere wenn alternative Verfahren zur systemischen Verabreichung zur Verfügung stehen.

Um die pharmakologischen Nachteile der oralen Verabreichung einerseits und die Nachteile der invasiven, rein mechanischen Durchdringung der Haut mit Hilfe medizinischer Instrumente (Injektionsnadeln, Skalpelle) andererseits zu umgehen, wurden Verfahren entwickelt, mit deren Hilfe der Wirkstoff kontinuierlich über eine bestimmte Zeitspanne durch die Haut hindurch diffundiert und so in den systemischen Blutkreislauf gelangt.

Die Haut stellt mit einer Fläche von etwa 20.000 cm² das größte Organ des menschlichen Körpers dar und erhält ungefähr ein Drittel der gesamten Blutversorgung im Organismus (vgl. Y. W, Chien, Logics of transdermal controlled drug administration. Drug Dev. Ind. Pharm. 1983, 9, 497). Sie übt in erster Linie Schutzfunktionen aus: Sie verhindert das Eindringen von Fremdstoffen und Mikroorganismen und den Verlust lebenswichtiger endogener Stoffe wie Wasser und Elektrolyte. Die Haut bildet jedoch keine völlig impermeable Barriere für exogene Stoffe, so dass Wirkstoffe über unterschiedliche Wege transkutan in den Organismus aufgenommen werden können. Maßgeblich beeinflusst wird die perkutane Permeabilität vom Applikationsort und von der Dicke der Hornschicht, welche die Hauptbarriere für Fremdstoffe darstellt. Während Hydrocortison als Modellsubstanz an Handflächen und Fußsohlen vermindert aufgenommen wird, ist die Aufnahmerate durch die Haut der Hinterohrregion und das Skrotum bis zu 40-fach erhöht verglichen mit dem Unterarm (vgl. H. Asche, Konzept und Aufbau transdermaler therapeutischer Systeme. Schweiz. Rundsch. Med. Prax., 1985, 74, 3).

Damit ein Wirkstoff transdermal wirken kann, muss er in ausreichender Menge durch die Epidermis der Haut diffundieren und vom Blutkreislauf aufgenommen werden. Die Epidermis übt dabei eine intensive Barrierefunktion aus, welche einerseits darauf zurückzuführen ist, dass der betreffende Wirkstoff nacheinander hydrophile, lipophile und dann wieder hydrophile Schichten passieren muss, andererseits aber auch der geringe Wassergehalt im *stratum corneum* die Diffusion von Wirkstoffen erschwert.

Die für eine systemische Wirkung und auch für die Wirkung der meisten äußerlich zu applizierenden Wirkstoffe erforderliche Permeation der Hornschicht erfolgt bei der unverletzten Haut transepidermal (interzellulär oder transzellulär) und durch Poren (transglandulär oder transfollikulär) (vgl. K. Karzel et al., Mechanismen transkutaner Resorption - Pharmakologische und biochemische Aspekte. Arzneim.-Forsch. Drug Res. 1989, 39, 1487)*.*

Begrifflich ist die *Penetration* eines Wirkstoffes von der *Permeation* eines Wirkstoffes zu unterscheiden: Penetration bedeutet, dass der Wirkstoff in die Haut hinein gelangt, während bei der Permeation der Wirkstoff durch die Haut hindurch bis in die Blutbahn gelangt. Zur systemischen Verabreichung von Wirkstoffen über den Blutkreislauf ist daher eine Permeation erforderlich.

Zur transdermalen Verabreichung wurden wirkstoffhaltige Pflaster entwickelt, mit deren Hilfe die Wirkstoffe in den systemischen Blutkreislauf gelangen können. Der Wirkstoff gelangt durch Diffusion in das unter der Haut liegende Gewebe und wird an die Blutgefäße abgeben, so dass er seine Wirksamkeit systemisch entfalten kann.

Wirkstoffhaltige Pflaster haben jedoch den Nachteil, dass sie für die Dauer der Verabreichung des Wirkstoffs spürbar und sichtbar auf der Haut aufgebracht sind. Ferner schafft die Abdeckfolie des Pflasters Okklusionsbedingungen. Die hierdurch bewirkte Quellung der Haut kann zu veränderten Diffusionsbedingungen für den Wirkstoff führen. Der Wirkstoff wird bei den meisten Pflastertypen unvollständig an die Haut abgegeben. Das Entfernen der Pflaster bereitet dem Patienten mitunter Schmerzen, da die Körperbehaarung an den Klebeflächen des Pflaster anhaftet und somit die Haare beim Abziehen des Pflasters mit den Haarwurzeln herausgerissen werden. Ferner haben transdermale Pflaster den Nachteil, dass die verwendeten Haftkleber bei den Patienten häufig Allergien und Hautreizungen verursachen. Auch das wiederholte Aufkleben und Abreißen der wirkstoffhaltigen Pflaster bei mehrfacher Anwendung an den gleichen Hautpartien kann mit der Zeit zu Hautrötungen und Irritationen führen.

Ein wesentlicher Nachteil von wirkstoffhaltigen Pflastern besteht auch darin, dass sie in ihrer Kontaktfläche zur Haut begrenzt sind. Da jedoch für die Wirksamkeit vieler Wirkstoffe vergleichsweise hohe Plasmakonzentrationen erforderlich sind, diese aber nur bei großflächiger Verabreichung des Wirkstoffs über die Haut zu erreichen sind, stoßen wirkstoffhaltige Pflaster schnell an ihre Grenzen. Dies ist insbesondere dann der Fall, wenn ein bestimmter Wirkstoff ein ungünstiges Permeationsverhalten zeigt, welches nur durch einen entsprechend großflächigen Hautkontakt kompensiert werden kann. Allgemein kann davon ausgegangen werden, dass Pflaster mit einer Größe von mehr als 50 cm² nur noch bedingt zur Anwendung geeignet sind. Da bei wirkstoffhaltigen Pflastern die Fläche des Systems begrenzt ist, können nur hochpotente Wirkstoffe zum Einsatz kommen, deren wirksame Plasmaspiegel im Bereich von ng/ml liegen. Bezüglich weiterer Einzelheiten kann beispielsweise auf K. H. Bauer et al, Lehrbuch der pharmazeutischen Technologie, Wissenschaftliche Verlagsgesellschaft mbH Stuttgart, 1999, 6. Auflage verwiesen werden.

Als Alternative zu den wirkstoffhaltigen Pflastern wurden wirkstoffhaltige Gele entwickelt, welche auf die Haut aufgetragen werden und binnen kurzer Zeit auf der Hautoberfläche eintrocknen. Die Trocknung erfolgt einerseits durch Verdunsten des Lösungsmittels, je nach Art des verwendeten Lösungsmittels ist es aber auch möglich, dass zumindest ein gewisser Anteil des Lösungsmittels unmittelbar nach dem Auftragen des Gels selektiv aus dem Gel in die Haut eindringt.

Wirkstoffhaltige Gele, welche eine Polymermatrix enthalten, zeichnen sich dadurch aus, dass nach dem Trocknen auf der Haut ein dünner Film aus der Polymermatrix zurückbleibt, worin der Wirkstoff und die anderen nicht-flüchtigen Bestandteile des Gels eingebettet sind. Im getrockneten Zustand kontrolliert die Polymermatrix die Diffusion des Wirkstoffs durch die Haut und ermöglicht somit eine gesteuerte Abgabe an den Organismus über eine längere Zeitspanne. Eine derart kontrollierte, zeitlich gesteuerte und kontinuierliche Abgabe des Wirkstoffs an den Blutkreislauf ist insbesondere bei Hormonen und Hormonderivaten wünschenswert, so dass sich diese wirkstoffhaltigen Gele insbesondere zur Verabreichung derartiger Wirkstoffe eignen. Durch die Umgehung des Gastrointestinaltraktes bei transdermaler Verabreichung wird auch der nachteilige First-Pass-Metabolismus umgangen.

Die Dosierung des Wirkstoffs lässt sich gut durch Variation der Gelmenge und der Fläche steuern, auf welcher das Gel aufgetragen wird. Die Wirkdauer von Wirkstoffen mit kurzer biologischer Halbwertszeit kann auf diese Weise verlängert werden. Bei Wirkstoffen mit geringer therapeutischer Breite kommt es zu einer Verringerung der Nebenwirkungen, häufig ist auch die Compliance der Patienten besser.

Wirkstoffhaltige Gele stellen echte einphasige Systeme dar. Es handelt sich um halbfeste Systeme, bei denen Flüssigkeiten durch Gelgerüstbildner verfestigt werden. Man unterscheidet, je nachdem ob die das Gel bildende Flüssigkeit Wasser oder ein Öl ist, Hydrogele und Oleogele. Die Hydrogele bestehen aus einer wässrigen Wirkstofflösung, die vorwiegend mit makromolekularen hydrophilen Substanzen zu einem Gel verfestigt wird. Als Gelgerüstbildner können organische Polymere, aber auch anorganische Substanzen, wie z.B. Bentonit und hochdisperses Siliciumdioxid eingesetzt werden. Oleogele sind Öle, die mit Gelgerüstbildnern versteift worden sind.

Hydrogele unterscheiden sich von Salben u.a. darin, dass Salben Zubereitungen sind, welche keine wässrige Phase enthalten. Hydrogele unterscheiden sich von Cremes darin, dass sie keine Lipidphase enthalten. Bezüglich weiterer Einzelheiten zur Abgrenzung der Hydrogele von anderen halbfesten Arzneiformen kann beispielsweise auf K. H. Bauer et al, Lehrbuch der pharmazeutischen Technologie, Wissenschaftliche Verlagsgesellschaft mbH Stuttgart, 1999, 6. Auflage verwiesen werden.

Gegenüber wirkstoffhaltigen Pflastern haben Gele den Vorteil, dass nach dem Trocknen auf der Haut lediglich ein dünner Film aus den Bestandteilen des Gels zurückbleibt, welche nicht sofort verdunsten bzw. in die Haut eindringen können. Durch die Trocknung entsteht ein sehr enger Kontakt zwischen der äußeren Hautschicht und dem Gelrückstand, auch Poren und feinste Unebenheiten auf der Haut werden durch das fluide Gel erreicht, was mit wirkstoffhaltigen Pflastern nur schwer zu bewerkstelligen ist. Dehnungen der Haut, welche durch Bewegungen des Patienten hervorgerufen werden, sind wegen der Elastizität und des Anhaftens des Gelrückstands über die gesamte Kontaktfläche unproblematisch.

Hingegen führen derartige Hautdehnungen bei wirkstoffhaltigen Pflastern häufig zu einer unerwünschten Lateralverschiebung der Kontaktfläche des Pflasters und der äußeren Hautschicht. Der Patient spürt die Wirkstoffaufnahme nicht und ist auch ansonsten in keiner Weise in seinen Körperbewegungen beeinträchtigt. Ferner können die Gele großflächig auf die Haut aufgetragen werden, so dass auch Wirkstoffe transdermal verabreicht werden können, welche für wirkstoffhaltige Pflaster ungeeignet sind. Dies ist ein wesentlicher Vorteil der Gele gegenüber den Pflastern. Nach beendeter Wirkstoffabgabe kann der dünne Filmrückstand, welcher auf der Haut verblieben ist, mit Wasser abgewaschen werden. Hautreizungen sind zwar in der Regel geringer als bei transdermalen Pflastern, sie stellen aber dennoch ein Problem bei vielen Hydrogelen dar, insbesondere da diese Gele zur Verbesserung der Permeation erhebliche Mengen (in derzeit verwendeten Hydrogelen ca. 70%) Ethanol aufweisen. Der hohe Ethanolgehalt ist notwendig, um eine gute Permeation zu gewährleisten, führt aber zu Hautreizungen.

Neben der Verträglichkeit und der guten Permeation werden an wirkstoffhaltige Gele auch bestimmte physikalische Anforderungen gestellt. Ihre Konsistenz muss so beschaffen sein, dass sie sich gut auf der Haut auftragen lassen und eine ausreichende Gelfestigkeit besitzen, damit das Gelgerüst erhalten bleibt, während die flüchtigen Bestandteile verdunsten bzw. in die Haut einziehen. Üblicherweise beträgt die Gelmenge, welche pro Dosiseinheit auf die Haut aufgetragen werden muss, 1-5 ml. Im Stand der Technik sind Polyacrylsäure-basierende Gele bekannt, welche bei Hautkontakt eine sofortige partielle Verflüssigung erfahren. Der Grund dafür ist die mangelnde Elektrolyttoleranz dieser Gelsysteme. Dieser Effekt führt bedingt durch Salze im Hydrolipidfilm auf der Haut zu einem schnellen Abrutschen bzw. Abtropfen der Produkte. Besondere Probleme ergeben sich auch, wenn die Hautoberfläche in Folge von Transpiration große Salzkonzentrationen enthält und feucht ist. Das problematische Applikationsverhalten ist besonders vor dem Hintergrund nachteilig, daß bei der Verabreichung von hochpotenten Steroidhormonen eine gezielte Anwendung am Bestimmungsort notwendig ist. Neben der Effektivität spielt hierbei auch die Sicherheit (Kontaminationsrisiko) eine Rolle.

Der ebenso im Stand der Technik bekannte Einsatz von Cellulose-Derivaten als Hydrogelbildner besitzt gegenüber den auf Polyacrylsäure-basierenden Gelen den Nachteil mangelnder sensorischer Eigenschaften sowie größerer Rückstände des Gelbildners auf der Haut. Die notwendigen Konzentrationen zur Ausbildung eines Gelgerüstes liegen 2-3fach höher gegenüber Polyacrylaten und verursachen einen sog. "Radiergummieffekt" (Aufriffeln) nach Anwendung auf der Haut. Diese Rückstände sind zur bestimmungsgemäßen Anwendung eines Hydrogels zur transdermalen Verabreichung von Wirkstoffen ebenso unerwünscht.

US 6,010,716 offenbart eine pharmazeutische Zusammensetzung zur transdermalen Verabreichung, welche eine Polymermatrix umfasst, die nach dem Trocknen einen flexiblen Film ausbildet. Die Polymermatrix ist ausgewählt aus Cellulose-Polymeren bzw. Cellulose-Copolymeren oder Vinylpyrrolidon/Vinylacetat-Copolymeren.

An die physikalischen und chemischen Eigenschaften von Wirkstoffen, die für eine transdermale Therapie mit Hilfe von Gelen in Frage kommen, sind verschiedene Anforderungen geknüpft. Das Molekulargewicht sollte unter 1000 gmol⁻¹ liegen. Die Substanz sollte lipidlöslich sein, aber auch eine gewisse Löslichkeit in wässrigen Medien aufweisen. Als Wirkstoffe, welche sich aufgrund ihrer physikalischen und chemischen Eigenschaften grundsätzlich zur transdermalen Verabreichung eignen, sind aus pharmakologischer Sicht Steroide besonders vorteilhaft. Es handelt sich dabei insbesondere um Steroide mit androgener Wirkung (Androgene).

Vielfach ist zum Erreichen der für die Wirkung ausreichenden Plasmakonzentration der Zusatz eines Permeationsvermittlers erforderlich. Viele Permeationsvermittler wurden für diesen Zweck untersucht und es kann beispielsweise auf E. W. Smith et al., Percutaneous Penetration Enhancers, CRC Press, 1995 verwiesen werden.

Verschiedene Zusammensetzungen zur transdermalen Verabreichung von Steroidhormonen, u.a. von bestimmten Androgenen (insbesondere von Testosteron) sind im Stand der Technik bekannt und werden beispielsweise in WO 96/08255, WO 97/03698, WO 97/43989, WO 98/37871, WO 99/13812, WO 00/71133, WO 02/066018 und A. W. Meikle et al, J. Clin. Endocrin. & Metab. 1992, 74, 623 offenbart. Die in den vorstehenden Druckschriften beschriebenen Zusammensetzungen zur transdermalen Verabreichung haben gemeinsam, dass das Steroidhormon in Kombination mit wenigstens einem Permeationsvermittler verabreicht wird, um die Permeation des Wirkstoffs durch die Haut überhaupt erst zu gewährleisten. Als Beispiele für bekannte Permeationsvermittler, welche für Testosteron beschrieben sind, können Fettsäuren, Fettsäureester mit einfachen Alkoholen, Fettsäuremonoester mit mehrwertigen Alkoholen, Fettalkohole und Terpene genannt werden.

Es besteht ein wesentlicher Unterschied zwischen wirkstoffhaltigen Pflastern und wirkstoffhaltigen Gelen, so dass nicht gefolgert werden kann, dass ein für ein wirkstoffhaltiges Pflaster geeigneter Permeationsvermittler auch für ein wirkstoffhaltiges Gel geeignet ist. Dies hängt unter anderem damit zusammen, dass wirkstoffhaltige Gele nach dem Auftragen auf der Haut eintrocknen, während in wirkstoffhaltigen Pflastern im Regelfall eine für Lösungsmittel undurchlässige Rückschicht ein Austrocknen verhindert.

Wirkstoffhaltige Pflaster enthalten gelegentlich in ihrem Innern einen gelierten Kern, in dem der Wirkstoff und einige Hilfsstoffe eingebettet sind. Diesbezüglich kann beispielsweise auf die Druckschrift EP-A 208 395 verwiesen werden. Derartige gelierte Kerne von wirkstoffhaltigen Pflastern sind jedoch in ihren Eigenschaften aus den oben genannten Gründen nicht mit wirkstoffhaltigen Gelen zu vergleichen, welche dazu bestimmt sind, auf der Haut aufgetragen zu werden.

Derzeit kommen in der Praxis nur solche wirkstoffhaltigen Gele zum Einsatz, die große Mengen an Ethanol (70 Gew.-% und mehr) enthalten, da angenommen wird, daß mit geringeren Mengen an Ethanol die erforderlichen Permeationsraten nicht erreicht werden können. Gele mit geringerem Ethanolgehalt sind zwar beschrieben, wegen ihrer geringen Permeationsraten aber nicht in der praktischen Anwendung.

Die mit dem hohen Ethanolgehalt einhergehenden Probleme wie Rötungen, Schwellungen und dauerhafte Schädigungen und Risse der Haut werden hingenommen. Im Stand der Technik geht man davon aus, dass niedermolekulare Alkylalkohole, wie z.B. Ethanol, die Fluidität der Flüssigkeiten im *stratum corneum* erhöhen oder Lipide aus dem *stratum corneum* extrahieren und somit die Permeation des Wirkstoffs durch die Haut begünstigen.

Die EP-A-0 573 133 offenbart Mittel zur transdermalen Applikation in Form eines Gels enthaltend Ethanol, 1,2-Propandiol , Isopropylmyristat, Hydroxypropylcellulose, Estradiol und Gestoden. Die in der EP-A-0 573 133 offenbarten Mittel enthalten jedoch keinen Kohlensäurediester.

Die WO 88/09185 A offenbart Kohlensäurediester, nämlich Propylencarbonat neben anderen penetrationsverstärkenden Mitteln. Jedoch offenbart diese Schrift nicht, das Propylencarbonat gegenüber den anderen in dem Dokument offenbarten penetrationsverstärekenden Mitteln besondere Eigenschaften hat, um die Permeation in pharmazeutischen Zusammensetzungen in Form eines Hydrogels zu verbessern.

EP-A 811 381 offenbart ein Gel, welches ein Estrogen und/oder ein Progestin, einen linearen aliphatischen primären Alkohol mit 11-19 Kohlenstoffatomen, einen Monoalkylether des Diethylenglykols, einen Alkohol mit 2-4 Kohlenstoffatomen, Glykol, Wasser, ein Polymer oder Copolymer aus Acrylsäure und ein tertiäres Amin enthält. Derartige Gele haben jedoch insbesondere den Nachteil, dass die Permeationseigenschaften für viele Wirkstoffe nicht vollständig zufriedenstellend sind. Die Permeationseigenschaften dieser Formulierungen können zwar verbessert werden, indem der Wassergehalt verringert und der Gehalt an Alkohol erhöht wird, dies führt aber notwendigerweise zu einer verringerten Verträglichkeit.

Ein Problem, das ebenfalls bei der Verwendung von transdermalen Gelen auftritt, besteht darin, dass pharmazeutische Zusammensetzungen in Form von Hydrogelen häufig nach dem Auftragen auf der Haut zerlaufen, wodurch das für die kontrollierte Abgabe des Wirkstoffs erforderliche Gelgerüst zerstört wird und durch mögliches Abtropfen des Produktes sowohl die Compliance des Patienten verschlechtert wird als auch die Wahrscheinlichkeit einer Kontamination nicht bestimmungsgemäßer Areale bzw. Gegenstände erhöht ist. Dieses Problem tritt bei normalem Hautzustand auf. In besonders starkem Maße tritt es dann auf, wenn die Hydrogele auf "schweißige" Haut aufgetragen werden, so dass bei bekannten und handelsüblichen Gelen gefordert wird, dass die Haut vor dem Auftragen des Gels gründlich gereinigt wird. Bevorzugt sollte ein Hydrogel aber auch intakt bleiben, wenn es auf Haut aufgetragen wird, die nicht vollständig von Schweiß befreit ist.

Eine Behandlung mit transdermalen Gelen ist häufig eine Langzeitherapie, insbesondere bei der Verabreichung von Steroiden. Für den Erfolg derartiger Behandlungen ist neben der effektiven Verabreichung des Wirkstoffs (einer hohen Permeationsrate) die Patientencompliance entscheidend. Falls ein Patient die Behandlung abbricht oder nicht regelmäßig durchführt, weil die Verabreichung des Gels als zu aufwendig angesehen wird (z.B. weil die Haut besonders gereinigt werden muß oder Kleidung verschmutzt werden kann) oder weil es zu Unverträglichkeiten kommt, wird der gesamte Behandlungserfolg in Frage gestellt.

Für eine Behandlung mit transdermalen Gelen ist neben einer hohen Permeationsrate auch eine möglichst lineare Permeation vorteilhaft, um über einen längeren Zeitraum einen möglichst konstanten Wirkstoffspiegel im Blut aufrechtzuerhalten.

Es besteht damit ein Bedarf an pharmazeutischen Zusammensetzungen, welche zur transdermalen Verabreichung von Wirkstoffen geeignet sind und Vorteile gegenüber den Zusammensetzungen des Standes der Technik aufweisen, insbesondere an Hydrogelen zur transdermalen Verabreichung von Wirkstoffen (sog. "transdermale Gele"), die eine ausgezeichnete Verträglichkeit mit sehr guten Permeationseigenschaften verbinden.

Für die erfindungsgemäße Verwendung zur Herstellung eines transdermalen Kontrazeptivums wird ein Hydrogel zur Verfügung gestellt, wie es in den Ansprüchen definiert ist und das eine ausgezeichnete Verträglichkeit mit einer hervorragenden Permeabilität kombiniert. In einer bevorzugten Ausführungsform kann das Hydrogel auch auf Haut mit ausgeprägtem Hydrolipidfilm aufgetragen werden, ohne dass es zum schnellen Zerlaufen und Abtropfen des Hydrogels kommt.

Es wurde überraschend gefunden, dass mit Hilfe einer pharmazeutischen Zusammensetzung in Form eines Hydrogels, welches einen Kohlensäurediester, einen C₂-C₄ Alkylalkohol, einen Wirkstoff und eine Polymermatrix enthält, Hautreizungen und andere Nebenwirkungen wirkungsvoll vermindert werden können. Gleichzeitig werden bei der erfindungsgemäßen Verwendung dieser Zusammensetzungen sehr gute Ergebnisse bezüglich des Permeationsverhaltens unterschiedlicher Wirkstoffe erzielt.

Pharmazeutische Zusammensetzungen, welche Kohlensäurediester, insbesondere Propylencarbonat enthalten, sind im Stand der Technik bekannt.

WO 98/10742 offenbart eine einphasige wasserfreie Zubereitung zur topischen Anwendung, welche Propylencarbonat, zumindest einen Alkohol, Glykol, Glycerin und einen therapeutisch oder kosmetisch wirksamen Inhaltsstoff enthält. Die Zusammensetzung ist vollständig wasserfrei und die Wirkstoffe können schnell in die Haut penetrieren. Eine Permeation ist allerdings nicht vorgesehen.

WO 00/41702 offenbart eine Zubereitung zur äußeren Anwendung, welche ein 21-Alkoxy-Steroid, Propylencarbonat und Polyoxyethylen/gehärtetes Castoröl enthält. Die Zusammensetzung eignet sich zur topischen Verabreichung von Steroiden zum Zwecke der Behandlung von Hautkrankheiten, wie z.B. chronischer oder akuter Ekzeme, atopischer Dermatitis, Kontaktdermatitis und Psoriasis.

JP 590 70 612 offenbart eine gelierte Salbengrundlage, welche ein Carboxyvinyl Polymer, Propylencarbonat, Propylenglykol, Polyethylenglykol und Ethanol enthält. Die Salbengrundlage kann Isopropyladipat enthalten, um die Penetration des Wirkstoffs in die Haut zu fördern. Auch hier soll keine Permeation stattfinden.

JP 91 94 396 offenbart eine Zusammensetzung, welche ein Antihistamin, ein Polymer auf Basis eines Aminoacrylats, ein säurelösliches Polymer und einen kurzkettigen Ester oder Ether mit insgesamt 4-20 Kohlenstoffatomen, wie z.B. Isopropylmyristat, Triacetin-Ethylenglykol-mononormalbutylether oder Propylencarbonat, umfasst. Die Zusammensetzung kann als Matrix für ein Pflaster verwendet werden.

US 3 924 004 offenbart eine Zusammensetzung zur topischen Anwendung, welche einen gesättigten Fettalkohol mit 16-24 Kohlenstoffatomen, Propylencarbonat, Glykol, ein Tensid, einen Plasticiser und Wasser enthält. Die Zusammensetzung kann zur topischen Verabreichung aller Arten von Wirkstoffen verwendet werden, insbesondere von entzündungshemmenden Corticosteroiden. Die Stabilität der Zusammensetzung ist verbessert, wenn die Zusammensetzung kein Wasser enthält.

EP-A 319 555 offenbart eine transdermal therapeutisch wirkende pharmazeutische Zubereitung, welche als Spray auf die Haut aufgetragen werden kann. Die Zubereitung enthält eine polymere flüssige zu einem flexiblen Film aushärtende Matrix, einen Wirkstoff, ein die Freigabe des Wirkstoffes steuerndes Lösungsmittel, in welchem der Wirkstoff zumindest teilweise löslich ist und ein auf der Haut verdampfendes Lösungsmittel für die Matrix. Als die Freigabe des Wirkstoffes steuerndes Lösungsmittel werden Sorbitanmacrogollaurat und/oder Paraffin und/oder mittelkettige Fettsäuredi- und/oder -triglyceride und/oder Propylencarbonat offenbart. Die Zubereitung ist wasserfrei.

Keine dieser Druckschriften offenbart eine pharmazeutische Zusammensetzung in Form eines Hydrogels, welche einen Kohlensäurediester, einen C₂-C₄ Alkylalkohol, einen Wirkstoff und eine Polymermatrix enthält. Die Verwendung von Kohlensäurediestern (z.B. von Propylencarbonat) als Permeationsvermittler für Wirkstoffe in pharmazeutischen Zusammensetzungen in Form von Hydrogelen, welche eine Polymermatrix und einen C₂-C₄ Alkylalkohol enthalten, ist im Stand der Technik noch nicht beschrieben.

Es wurde überraschend gefunden, dass bei Verwendung eines Kohlensäurediesters als Permeationsvermittler für den Wirkstoff der Gehalt des C₂-C₄ Alkylalkohols in der Zusammensetzung relativ gering gehalten werden kann, ohne dabei negative Auswirkungen auf das Permeationsverhalten zu haben. Dem Kohlensäurediester kommt innerhalb der Zusammensetzung besondere Bedeutung zu, da sofort nach dem Auftragen der pharmazeutischen Zusammensetzung das Wasser und der C₂-C₄ Alkylalkohol weitgehend verdunsten bzw. in die Haut einziehen, während der schlechter flüchtige Kohlensäurediester zusammen mit dem Wirkstoff in der Polymermatrix zurückbleibt und somit maßgeblich das pharmakokinetische Verhalten des Wirkstoffs beeinflusst.

Es wurde gefunden, dass Kohlensäurediester besonders verträglich und chemisch stabil sind, im Regelfall keine allergischen Reaktionen hervorrufen und mit den in der pharmazeutischen Zusammensetzung enthaltenen zusätzlichen Inhaltsstoffen, insbesondere mit dem im Hydrogel enthaltenen Wasser und dem C₂-C₄ Alkylalkohol gut zusammenwirken. Der Gewichtsanteil des Kohlensäurediesters, des in der Zusammensetzung enthaltenen Wassers und des C₂-C₄ Alkylalkohols kann derart gewählt werden, dass die Menge des C₂-C₄ Alkylalkohols und die durch diesen verursachten Nebenwirkungen minimiert werden, gleichzeitig aber dennoch sehr gute Permeationseffizienzen für den Wirkstoff erreicht werden.

Handelt es sich bei dem erfindungsgemäßen Kohlensäurediester um eine chirale Verbindung, so liegt diese bevorzugt in racemischer Form vor. Es ist aber auch möglich, dass die erfindungsgemäße Zusammensetzung den Kohlensäurediester in angereicherter Form eines Enantiomers bzw. Diastereomers enthält.

Bevorzugt weist der Kohlensäurediester ein Molekulargewicht von weniger als 750 gmol⁻¹, bevorzugter von weniger als 500 gmol⁻¹, insbesondere von weniger als 250 gmol⁻¹ auf. Bevorzugt enthält der Kohlensäurediester nicht mehr als 12, bevorzugter nicht mehr als 10, bevorzugter nicht mehr als 7, insbesondere nicht mehr als 5 Kohlenstoffatome.

In einer erfindungsgemäß bevorzugten Ausführungsform ist der Kohlensäurediester eine Verbindung der allgemeinen Formel (I) 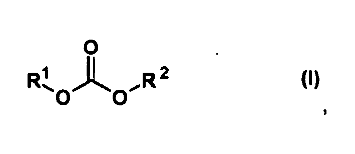 worin entweder R¹ und R² unabhängig voneinander C₁-C₆-Alkyl, C₂-C₆-Alkenyl, C₂-C₆-Alkinyl, C₄-C₆-Cycloalkyl, C₁-C₆-Heterocycloalkyl, Phenyl, C₁-C₆-Heteroaryl, Phenyl-C₁-C₄-alkyl oder C₂-C₁₀-Heteroarylalkyl sind, wobei die Alkyl-, Alkenyl- und Alkinyl-Gruppen gegebenenfalls jeweils bis zu dreimal durch Sauerstoffatome und/oder Schwefelatome unterbrochen sein können, oder R¹ und R² unabhängig voneinander eine der oben genannten Bedeutungen haben und über eine C-C-Bindung miteinander verbunden sind. Sind R¹ und R² über eine C-C-Bindung miteinander verbunden, so handelt es sich bei der Verbindung der allgemeinen Formel (I) um einen cyclischen Kohlensäurediester. Die C₁-C₆-Heterocycloalkyl-, C₁-C₆-Heteroaryl- und C₂-C₁₀-Heteroarylalkyl-Gruppen können im Heterocyclus 1 bis 4 Heteroatome enthalten, welche unabhängig voneinander ausgewählt sind aus N, O und S.

Bevorzugte Reste R¹ und R² (bei denen die Alkylgruppen gegebenenfalls bis zu dreimal durch Sauerstoffatome und/oder Schwefelatome unterbrochen sind) werden im folgenden aufgeführt:
C₁-C₆-Alkyl: -CH₃, -CH₂CH₃, -CH₂CH₂CH₃, -CH(CH₃)₂, -CH₂CH₂CH₂CH₃, -CH(CH₃)CH₂CH₃, -CH₂CH(CH₃)CH₃, -C(CH₃)₃, -CH₂OCH₃, -CH₂SCH₃, -CH₂CH₂OCH₃, -CH₂CH₂SCH₃, - CH₂CH₂OCH₂CH₃, -CH₂CH₂SCH₂CH₃ und -CH₂CH₂OCH₂CH₂OCH₂CH₃;
C₂-C₆-Alkenyl: -CH=CH₂, -CH₂CH=CH₂, -CH₂CH=CHCH₃, -CH(CH₃)CH=CH₂, und -CH=C(CH₃)₂;
C₂-C₆-Alkinyl: -C≡CH, -CH₂C≡CH und -CH₂C≡CCH₃;
C₄-C₆-Cycloalkyl: -Cyclopentyl und -Cyclohexyl;
C₁-C₆-Heterocycloalkyl: -Piperidyl, -Morpholinyl, -Tetrahydropyranyl und Furanyl;
C₁-C₆-Heteroaryl: -Pyridyl, -Pyrrolyl und -Imidazolyl;
Phenyl-C₁-C₄-alkyl: -CH₂-Phenyl, -CH₂CH₂-Phenyl, -CH₂CH₂O-Phenyl, - CH₂CH₂OCH₂-Phenyl und -CH₂CH₂OCH₂CH₂O-Phenyl;
C₂-C₁₀-Heteroarylalkyl: -CH₂-Pyridyl, -CH₂CH₂-Pyridyl, -CH₂CH₂OCH₂-Pyridyl, - CH₂CH₂-Imidazolyl, -CH₂CH₂O-Imidazolyl und -CH₂CH₂OCH₂-Imidazolyl.

Als bevorzugte Reste R¹ und R², welche über eine C-C-Bindung miteinander verbunden sind, können die folgenden bivalenten Gruppen genannt werden:
-CH₂CH₂-, -CH=CH-, -CH(CH₃)CH₂-, -CH(OCH₃)CH₂-, -CH(OCH₂CH₃)CH₂-,
-CH(CH₃)CH(CH₃)-, -CH(OCH₃)CH(OCH₃)-, -CH₂CH₂CH₂-, -CH(CH₃)CH₂CH₂-,
-CH₂CH(CH₃)CH₂-, -CH(CH₃)CH₂CH(CH₃)- und -CH₂CH₂OCH₂CH₂-.

Als Permeationsvermittler ist es besonders bevorzugt, dass der Kohlensäurediester eine Verbindung der allgemeinen Formel (II) 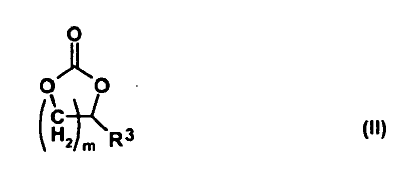 ist, worin der Index m eine Zahl von 1 bis 3 und R³ Wasserstoff oder C₁-C₄-Alkyl ist.

Erfindungsgemäß besonders bevorzugt ist ein Kohlensäurediester der allgemeinen Formel (II), bei dem der Index m 1 und R³ entweder Methyl (Propylencarbonat) oder Wasserstoff (Ethylencarbonat) ist.

Propylencarbonat [(±)-4-Methyl-1,3-dioxolan-2-on] hat ein Molekulargewicht von 102 gmol⁻¹ und einen Siedepunkt von 242°C. Es kommt in zwei enantiomeren Formen vor. Grundsätzlich kann die erfindungsgemäße Zusammensetzung Propylencarbonat in Reinform des R-Enantiomers oder des S-Enantiomers oder eines der beiden Enantiomere in angereicherter Form enthalten. Erfindungsgemäß bevorzugt ist es, dass Propylencarbonat als Racemat in der Zusammensetzung enthalten ist.

Bevorzugt beträgt der Gewichtsanteil des Kohlensäurediesters an der pharmazeutischen Zusammensetzung 1,0-40,0 Gew.-%, bevorzugter 2,5-30,0 Gew.-%, noch bevorzugter 5,0-20,0 Gew.-%, insbesondere 7,5-12,5 Gew.-%.

Kohlensäurediester sind im Stand der Technik bekannt. Sie können beispielsweise durch die Umsetzung von Alkoholen mit Phosgen oder Phosgen-Derivaten erhalten werden. Propylencarbonat kann technisch beispielsweise durch Umsetzung von 1,2-Propylenglykol mit Phosgen erhalten werden. Zahlreiche Kohlensäurediester sind kommerziell im Handel erhältlich.

Die erfindungsgemäß verwendete Zusammensetzung enthält bevorzugt einen C₂-C₄ Alkylalkohol ausgewählt aus der Gruppe bestehend aus Ethanol, n-Propanol und isoPropanol. Besonders bevorzugt ist Ethanol.

Bevorzugt beträgt der Gewichtsanteil des C₂-C₄ Alkylalkohols an der pharmazeutischen Zusammensetzung 25,0-70,0 Gew.-%, bevorzugter 30,0-65,0 Gew.-%, noch bevorzugter 30,0-60,0 Gew.-%, insbesondere 40,0-60,0 Gew.-%.

In einer bevorzugten Ausführungsform beträgt das relative Gewichtsverhältnis zwischen dem Kohlensäurediester und dem C₂-C₄ Alkylalkohol 0,01-1,50, bevorzugter 0,08-0,80, insbesondere 0,10-0,30.

In einer besonders bevorzugten Ausführungsform enthält die erfindungsgemäß verwendete Zusammensetzung Ethanol in Kombination mit Ethylencarbonat oder Propylencarbonat, wobei die Kombination von Ethanol mit Propylencarbonat bevorzugter ist.

Die erfindungsgemäß verwendete Zusammensetzung eignet sich grundsätzlich zur transdermalen Verabreichung verschiedenster Wirkstoffe, bevorzugt enthält die Zusammensetzung Steroide als Wirkstoff.

In einer bevorzugten Ausführungsform enthält die erfindungsgemäß verwendete Zusammensetzung als Wirkstoff eine Verbindung der allgemeinen Formel (III) 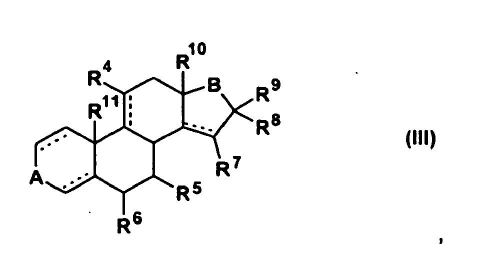 worin
R⁴ Wasserstoff, Fluor, Chlor, C₁-C₃-Alkyl oder eine gegebenenfalls acetylierte Hydroxylgruppe ist,
R⁵, R⁶, R⁷, R⁸, R⁹, R¹⁰ und R¹¹ unabhängig voneinander Wasserstoff oder C₁-C₃-Alkyl sind,
die gestrichelten Linien unabhängig voneinander eine optionale Bindung sind und A und B unabhängig voneinander eine Carbonylgruppe oder eine Gruppe 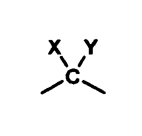 sind, worin X eine Hydroxylgruppe oder deren Ester einer Carbonsäure mit 1-8 Kohlenstoffatomen und Y Wasserstoff oder C₁-C₃-Alkyl ist. Erfindungsgemäß bevorzugt sind Verbindungen der allgemeinen Formel (III), worin R⁷, R⁸ und R⁹ Wasserstoff sind. Verbindungen der allgemeinen Formel (III) sind im Stand der Technik bekannt. Es kann beispielsweise auf die Druckschriften DE 1 182 229, US 3,341,557, US 4,000,273, WO 99/26962, WO 02/48169, Hill et al., Dictionary of Steroids, Chapman and Hall, 1991*,* Fieser & Fieser, Steroide, VCH Weinheim, 1961*,* J.F. Griffin et al., Atlas of Steroid Structure, Plenum Pub Corp, 1984 und G.W.A. Milne, Ashgate Handbook of Endocrine Agents and Steroids, Ashgate Publishing Company, 2000 verwiesen werden.

In einer besonders bevorzugten Ausführungsform enthält die erfindungsgemäß verwendete Zusammensetzung als Wirkstoff eine Verbindung der allgemeinen Formel (IV) 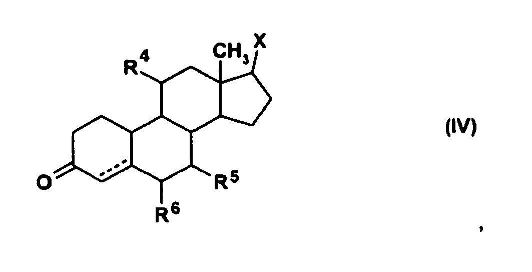 worin X eine Hydroxylgruppe oder deren Ester einer Carbonsäure mit 1-4 Kohlenstoffatomen, R⁴ Wasserstoff, Fluor oder eine Hydroxylgruppe, R⁵ und R⁶ unabhängig voneinander Wasserstoff, Methyl oder Ethyl sind und die gestrichelte Linie eine optionale Bindung ist. Bei den Verbindungen der allgemeinen Formel (IV) handelt es sich um 19-Nor-Androgen-Derivate, d.h. die üblicherweise am Kohlenstoffatom 19 enthaltene Methylgruppe ist durch Wasserstoff ersetzt. Diese Steroide zeichnen sich häufig durch eine besondere pharmakologische Wirksamkeit aus.

Die Verbindungen der allgemeinen Formel (III) bzw. (IV) können in Reinform oder als Gemisch mehrerer Stereoisomere vorliegen. Bevorzugt liegen die Verbindungen der allgemeinen Formel (III) bzw. (IV) in Form der im wesentlichen reinen Stereoisomere vor, d.h. die ee- bzw. de- Werte liegen bevorzugt oberhalb von 90%, bevorzugter oberhalb von 95%, insbesondere oberhalb von 99%.

Erfindungsgemäß besonders bevorzugt ist es, wenn die Zusammensetzung als Wirkstoff eine Verbindung der allgemeinen Formel (V) 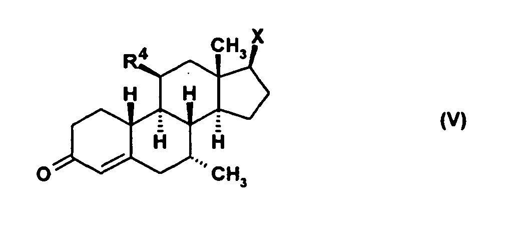 enthält, worin R⁴ Fluor oder Wasserstoff und X eine Hydroxylgruppe oder deren Essigsäureester ist. Besonders bevorzugt ist X eine Hydroxylgruppe.

Ist R⁴ Wasserstoff und X eine Hydroxylgruppe, so handelt es sich bei der Verbindung um 7α-Methyl-19-nortestosteron (MENT). Ist R⁴ Wasserstoff und der Rest X der Essigsäureester einer Hydroxylgruppe, so handelt es sich bei der Verbindung um das entsprechende Acetat (MENTAc). Ist R⁴ Fluor und X eine Hydroxylgruppe, so handelt es sich bei der Verbindung um 7α-Methyl-11β-fluor-19-nortestosteron (eF-MENT). Ist R⁴ Fluor und der Rest X der Essigsäureester einer Hydroxylgruppe, so handelt es sich bei der Verbindung um das entsprechende Acetat (eF-MENTAc).

Diese Verbindungen sind im Stand der Technik bekannt, es kann auf Sundaram et al., Annals in Medicine, 1993, 25, 199 und auf WO 2002/59139 A1 verwiesen werden.

Bevorzugt beträgt der Gewichtsanteil der Verbindung der allgemeinen Formel (III), (IV) bzw. (V) an der pharmazeutischen Zusammensetzung 0,001-10,0 Gew.-%, bevorzugter 0,01-5,0 Gew.-%, noch bevorzugter 0,1-2,5 Gew.-%, insbesondere 0,5-1,0 Gew.-%.

Das relative Gewichtsverhältnis der Verbindungen der allgemeinen Formel (III), (IV) oder (V) zum Kohlensäurediester beträgt bevorzugt 0,0001-10, bevorzugter 0,005-1, insbesondere 0,05-0,1.

In einer besonders bevorzugten Ausführungsform der Erfindung enthält die pharmazeutische Zusammensetzung als Wirkstoff eine Verbindung der allgemeinen Formel (V), als C₂-C₄ Alkylalkohol Ethanol und als Kohlensäurediester Ethylencarbonat oder Propylencarbonat, wobei die Kombination von eF-MENT mit Ethanol und Propylencarbonat besonders bevorzugt ist.

In einer bevorzugten Ausführungsform enthält die erfindungsgemäß verwendete Zusammensetzung als Wirkstoff eine Kombination aus zwei oder mehreren Verbindungen der allgemeinen Formeln (III), (IV) oder (V).

Es ist ebenfalls möglich, daß die erfindungsgemäß verwendete Zusammensetzung als Wirkstoff eine oder mehrere Wirkstoffe enthält, die von den Verbindungen der Formeln (III), (IV) oder (V) verschieden sind. Als Beispiele für derartige Wirkstoffe können Androgene, Antiandrogene, 5α-Reduktasehemmer, Estrogenrezeptor-Modulatoren, Estrogene, Antiestrogene, Gestagene, Antigestagene, uteruswirksame Substanzen, m-Cholinozeptor-Antagonisten, Prostaglandine bzw. Prostaglandin-Derivate und/oder Nicotin genannt werden.

In einer bevorzugten Ausführungsform enthält die erfindungsgemäß verwendete Zusammensetzung eine Kombination aus einem oder mehreren Wirkstoffen der allgemeinen Formel (III), (IV) oder (V) und einem oder mehreren Wirkstoffen, die von den Verbindungen der Formeln (III), (IV) oder (V) verschieden sind. Als Beispiele für derartige Wirkstoffe, die von den Verbindungen der Formeln (III), (IV) oder (V) verschieden sind, können Androgene, Antiandrogene, 5α-Reduktasehemmer, Estrogenrezeptor-Modulatoren, Estrogene, Antiestrogene, Gestagene, Antigestagene, uteruswirksame Substanzen, m-Cholinozeptor-Antagonisten, Prostaglandine bzw. Prostaglandin-Derivate und/oder Nicotin genannt werden.

In einer bevorzugten Ausführungsform enthält die erfindungsgemäß verwendete Zusammensetzung als Wirkstoff ein oder mehrere Androgene. Als Beispiele können genannt werden MENT, MENTAc, eF-MENT, eF-MENTAc, Testosteron, Testosteronpropionat, Testosteronundecanoat, Testosteronenantat, Mesterolon, Nandrolondecanoat, Clostebolacetat oder Metenolonacetat.

In einer bevorzugten Ausführungsform enthält die erfindungsgemäß verwendete Zusammensetzung als Wirkstoff ein oder mehrere Antiandrogene, wie z.B. Cyproteronacetat, Flutamid oder Bicalutamid.

In einer bevorzugten Ausführungsform enthält die erfindungsgemäß verwendete Zusammensetzung als Wirkstoff ein oder mehrere 5α-Reduktasehemmer, wie z.B. Finasterid oder 17α-Estradiol.

In einer bevorzugten Ausführungsform enthält die erfindungsgemäß verwendete Zusammensetzung als Wirkstoff ein oder mehrere selektive Estrogenrezeptor-Modulatoren, wie z.B. Raloxifen.

In einer bevorzugten Ausführungsform enthält die erfindungsgemäß verwendete Zusammensetzung als Wirkstoff ein oder mehrere Estrogene, wie z.B. Estradiol, Estradiolvalerat oder Estriol.

In einer bevorzugten Ausführungsform enthält die erfindungsgemäß verwendete Zusammensetzung als Wirkstoff ein oder mehrere konjugierte Estrogene, Estrogensulfamate oder Antiestrogene, wie z.B. Clomifen oder Taxol oder partielle Antiestrogene wie z.B. Raloxifen.

In einer bevorzugten Ausführungsform enthält die erfindungsgemäß verwendete Zusammensetzung als Wirkstoff ein oder mehrere Gestagene, wie z.B. Progesteron, Hydroxyprogesteroncapronat, Megestrolacetat, Medroxyprogesteronacetat, Chlormadinonacetat, Cyproteronacetat, Medrogeston, Dydrogesteron, Norethisteron, Norethisteronacetat, Norethisteronenantat, Levonorgestrel, Gestoden, Etonogestrel, Dienogest, Danazol, Norgestimat, Lynestrenol, Desogestrel oder Drospirenon.

In einer bevorzugten Ausführungsform enthält die erfindungsgemäß verwendete Zusammensetzung als Wirkstoff ein oder mehrere Antigestagene, wie z.B. Mifepriston oder Mesoprogestin.

In einer bevorzugten Ausführungsform enthält die erfindungsgemäß verwendete Zusammensetzung als Wirkstoff ein oder mehrere uteruswirksame Substanzen, wie z.B. Oxytocin.

In einer bevorzugten Ausführungsform enthält die erfindungsgemäß verwendete Zusammensetzung als Wirkstoff ein oder mehrere m-Cholinozeptor-Antagonisten, wie z.B. Scopolamin.

In einer bevorzugten Ausführungsform enthält die erfindungsgemäß verwendete Zusammensetzung als Wirkstoff ein oder mehrere Prostaglandine bzw. Prostaglandin-Derivate, wie z.B. Alprostadil, Gemeprost, Dinoproston, Sulproston, Dinoprost, Latanoprost oder Misoprostol.

In einer bevorzugten Ausführungsform enthält die erfindungsgemäß verwendete Zusammensetzung als Wirkstoff Nicotin.

Auch eine Verwendung einer Kombination aus zwei oder mehreren der vorstehenden Wirkstoffe in der Kontrazeption ist bevorzugt.

Die erforderliche Dosierung der Androgene, Antiandrogene, 5α-Reduktasehemmer, Estrogenrezeptor-Modulatoren, Estrogene, Antiestrogene, Gestagene, Antigestagene, uteruswirksamen Substanzen, m-Cholinozeptor-Antagonisten, Prostaglandine bzw. Prostaglandin-Derivate und des Nicotins ist dem Fachmann bekannt. In diesem Zusammenhang kann beispielsweise auf Mutschler, Arzneimittelwirkungen - Lehrbuch der Pharmakologie und Toxikologie, 2001 *und* W. Forth et al., Allgemeine und Spezielle Pharmakologie und Toxikologie, BI Wissenschaftsverlag 1992, 6. Auflage verwiesen werden.

In einer bevorzugten Ausführungsform enthält die erfindungsgemäß verwendete Zusammensetzung eine Kombination mehrerer Wirkstoffe, ausgewählt aus der Gruppe bestehend aus Androgenen und/oder Antiandrogenen und/oder 5α-Reduktasehemmern und/oder Estrogenrezeptor-Modulatoren und/oder Estrogenen und/oder Antiestrogenen und/oder Gestagenen und/oder Antigestagenen und/oder uteruswirksamen Substanzen und/oder m-Cholinozeptor-Antagonisten und/oder Prostaglandinen bzw. Prostaglandin-Derivaten und/oder Nicotin und/oder Verbindungen der allgemeinen Formel (III), (IV) und/oder (V).

Bevorzugt enthält die erfindungsgemäß verwendete Zusammensetzung weitere Inhaltsstoffe als Hilfsstoffe.

In einer bevorzugten Ausführungsform enthält die Zusammensetzung als Hilfsstoffe Cyclomethicon und/oder Isopropylmyristat. Im Hydrogel dienen Cyclomethicon und Isopropylmyristat einer guten Spreitung und der Pflege auf der Haut. In einer bevorzugten Ausführungsform enthält die Zusammensetzung als Hilfsstoff Glycerin. Glycerin dient als Feuchthalter für die Zusammensetzung und die Haut.

In einer bevorzugten Ausführungsform enthält die Zusammensetzung als Hilfsstoffe Cyclomethicon, Isopropylmyristat und Glycerin. Der Wasseranteil in der Zusammensetzung und die Hilfsstoffe bewirken in ihrer Kombination eine bessere Verträglichkeit der Zusammensetzung. Die Konzentration der pflegenden Hilfsstoffe (Cyclomethicon und Isopropylmyristat), sowie die Flüchtigkeit des Cyclomethicons sind von Bedeutung, da Rückstände der Zusammensetzung auf der Haut weitgehend vermieden werden sollten - durch Hautkontakt des Patienten mit einer anderen Person könnte diese sonst kontaminiert werden.

Als weitere Hilfsstoffe können Polyethylenglykol und/oder flüchtige Siliconöle, wie z.B. Hexamethylcyclotrisiloxan, Octamethylcyclotetrasiloxan und/oder Decamethylcyclopentasiloxan in der erfindungsgemäßen Zusammensetzung enthalten sein. Als Hilfsstoffe können neben Wasser und dem C₂-C₄ Alkylalkohol beispielsweise auch die Lösungsmittel Benzylalkohol, Dimethylformamid oder Dimethylsulfoxid verwendet werden.

Bevorzugt sind auch mehrwertige Alkohole, wie Ethylenglykol, Propylen-glykol, Butylenglykol oder Hexylenglykol. Um eine Austrocknung der Zusammen-setzung zu verhindern, können neben oder anstelle von Glycerin Sorbit, Mannit, Polyethylenglykol und/oder Polypropylenglykol bzw. ein Copolymer aus Ethylenglykol und Propylenglykol zugesetzt werden. Glycerin hat sich als besonders geeignet erwiesen. Weiterhin kann die pharmazeutische Zusammensetzung beispielsweise Farbstoffe, Geruchsstoffe, Antioxidatien, Tenside, Bakterizide, Fungizide, Komplexbildner, Cyclodextrine, Elektrolyte und/oder Viskositätsverbesserer enthalten. Deratige Hilfsstoffe sind dem Fachmann bekannt. Bezüglich weiterer Informationen kann beispielsweise auf Fiedler, Lexikon der Hilfsstoffe, ECU Aulendorf 1996, 4. Auflage *und* Hunnius Studienausgabe, de Gruyter 1993, 7. Auflage, verwiesen werden.

Die Hilfsstoffe verbessern die Verträglichkeit der Zusammensetzung auf der Haut. Bevorzugt beträgt der relative Anteil der Hilfsstoffe an der pharmazeutischen Zusammensetzung 0,001-15,0 Gew.-%, bevorzugter 0,01-10,0 Gew.-%, noch bevorzugter 0,5 bis 5,0 Gew.-%, insbesondere 1,0 bis 4,0 Gew.-%. Ein Anteil von 1,0-2,0 Gew.-% Cyclomethicon und/oder 0,3-0,8 Gew.-% Isopropylmyristat und/oder 0,5-1,5 Gew.-% Glycerin ist besonders bevorzugt.

Bei der erfindungsgemäß verwendeten pharmazeutischen Zusammensetzung handelt es sich um ein Hydrogel. Der Wassergehalt des Hydrogels beträgt bevorzugt 5,0-90,0 Gew.-%, bevorzugter 10,0-70,0 Gew.-%, noch bevorzugter 20,0-50,0 Gew.-%, insbesondere 25,0-40,0 Gew.-%.

Das relative Gewichtsverhältnis des C₂-C₄ Alkylalkohols zum Wasser beträgt bevorzugt 0,1-10,0, bevorzugter 0,5-5,0, noch bevorzugter 1,0-3,0, insbesondere 1,8-2,2.

In einer bevorzugten Ausführungsform der Erfindung enthält die pharmazeutische Zusammensetzung als Kohlensäurediester entweder Propylencarbonat oder Ethylencarbonat und als C₂-C₄ Alkylalkohol Ethanol. Für diesen Fall enthält die pharmazeutische Zusammensetzung bevorzugt 29,0-73,0 Gew.-% Ethanol und 5,0-50,0 Gew.-% Wasser, bevorzugter 34,0-68,0 Gew.-% Ethanol und 10,0-45,0 Gew.-% Wasser, noch bevorzugter 39,0-63,0 Gew.-% Ethanol und 15,0-40,0 Gew.-% Wasser, besonders bevorzugt 44,0-58,0 Gew.-% Ethanol und 20,0-35,0 Gew.-% Wasser und insbesondere 54,8-57,5 Gew.-% Ethanol und 27,2-30,8 Gew.-% Wasser.

Der pH-Wert der erfindungsgemäß verwendeten pharmazeutischen Zusammensetzung wird bevorzugt auf einen Wert zwischen 4,5 und 7,5, bevorzugter zwischen 5,0 und 7,0, insbesondere zwischen 5,5 und 6,5 eingestellt. Dazu eignen sich Puffer, wie z.B. Tris-(hydroxymethyl)-aminomethan, Triethanolamin oder Basen, wie z.B. Diisopropylamin oder Kaliumhydroxid. Auch weitere geeignete Puffersubstanzen und Basen sind dem Fachmann bekannt. Bezüglich weiterer Informationen kann beispielsweise auf Fiedler, Lexikon der Hilfsstoffe, ECU Aulendorf 1996, 4. Auflage *und* Hunnius Studienausgabe, de Gruyter 1993, 7. Auflage verwiesen werden.

Die erfindungsgemäß verwendete pharmazeutische Zusammensetzung enthält ferner eine Polymermatrix. Diese Polymermatrix umfasst zumindest einen Gelgerüstbildner und gegebenenfalls ein oder mehrere Verdickungsmittel, wodurch die rheologischen Eigenschaften der Zusammensetzung verbessert werden. Durch das Gelgerüst steigt die Compliance der Patienten deutlich an, was ein wesentlicher Vorteil der erfindungsgemäßen Zusammensetzungen ist. Eine Balance der Geleigenschaften ist wichtig, welche durch die erfindungsgemäße Zusammensetzung gewährleistet ist. In der Regel erfolgt eine kontinuierliche Anwendung der erfindungsgemäßen Zusammensetzung durch den Patienten, was jedoch insbesondere wegen der einfachen Widerauftragbarkeit des Hydrogels auf der Haut keine Schwierigkeiten bereitet.

Geeignete Gelgerüstbildner sind im Stand der Technik bekannt. Erfindungsgemäß bevorzugt umfasst die Polymermatrix als Gelgerüstbildner ein Acrylpolymer. Das Acrylpolymer kann ein Homopolymer oder ein Copolymer sein.

Handelt es sich bei dem Acrylpolymer um ein Homopolymer, so leitet sich dieses bevorzugt von einem Acrylsäure-C₁-C₃₀-alkylester oder einem Methacrylsäure-C₁-C₃₀-alkylester ab.

Handelt es sich bei dem Acrylpolymer um ein Copolymer, so leitet sich dieses bevorzugt von Acrylsäure, Methacrylsäure, Acrylsäure-C₁-C₃₀-alkylester oder Methacrylsäure-C₁-C₃₀-alkylester in Kombination mit einem oder mehreren Vinylmonomeren ab. Das oder die Vinylmonomere können entweder ebenfalls Acrylsäure, Methacrylsäure, ein Acrylsäure-C₁-C₃₀-alkylester oder ein Methacrylsäure-C₁-C₃₀-alkylester sein, es können aber auch beispielsweise Styrol, Ethylen, Propylen, Vinylchlorid, Vinylidenchlorid, Tetrafluorethylen, Vinylacetat, Vinylether oder Vinylpyrrolidon im Copolymer enthalten sein.

Als Acrylpolymere sind insbesondere Homopolymere oder Copolymere bevorzugt, welche durch Polymerisation von Acrylsäure, Methacrylsäure, Acrylsäure-C₁-C₃₀-alkylester und/oder Methacrylsäure-C₁-C₃₀-alkylester erhalten werden.

Die in der erfindungsgemäß verwendeten Zusammensetzung enthaltenen Acrylpolymere können unvernetzt oder vernetzt sein. In einer bevorzugten Ausführungsform enthält die erfindungsgemäße Zusammensetzung ein vernetztes Acrylpolymer.

Handelt es sich bei dem Acrylpolymer um ein unvernetztes Polymer, so liegt das gewichtsmittlere Molekulargewicht M_{w} des Acrylpolymers bevorzugt im Bereich von 50.000 bis 2.500.000 gmol⁻¹, bevorzugter im Bereich von 100.000 bis 2.000.000 gmol⁻¹, insbesondere im Bereich von 500.000 bis 1.500.000 gmol⁻¹.

Besonders bevorzugt enthält die erfindungsgemäß verwendete Zusammensetzung als Gelgerüstbildner Acrylpolymere, welche Copolymere sind und sich von einer Mischung aus Acrylsäure und Acrylsäure-C₁₀-C₃₀-alkylestern ableiten. In einer besonders bevorzugten Ausführungsform sind diese Copolymere quervernetzt, beispielsweise mit Allylpentaerythrit. Derartige quervernetzte Polymere sind im Stand der Technik bekannt. Beispielsweise ist ein Acrylat-C₁₀-C₃₀-alkylacrylat unter der Bezeichnung Pemulen^{®} TR1 kommerziell erhältlich. Es handelt sich um ein Acrylat/C10-30 Alkylacrylat Crosspolymer. Die allgemeine chemische Struktur von Pemulen^{®} TR1 kann vereinfacht wie folgt dargestellt werden: 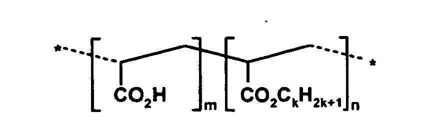 mit k = 10-30.

Bevorzugt beträgt der Gewichtsanteil des Gelgerüstbildners an der pharmazeutischen Zusammensetzung 0,001-20,0 Gew.-%, bevorzugter 0,005-10,0 Gew.-%, noch bevorzugter 0,01-5,0 Gew.-%, insbesondere 0,5-1,0 Gew.-%.

Neben dem Gelgerüstbildner kann die Polymermatrix der erfindungsgemäßen Zusammensetzung weitere Polymere enthalten. Diese können als Verdickungsmittel wirken. Erfindungsgemäß bevorzugt enthält die Zusammensetzung als Verdickungsmittel Polyacrylsäure. Polyacrylsäure wird kommerziell beispielsweise unter der Bezeichnung Carbopol^{®} vermarktet. Erfindungsgemäß ist Carbopol^{®} 980 besonders bevorzugt.

Bevorzugt beträgt der Gewichtsanteil der Polyacrylsäure an der pharmazeutischen Zusammensetzung 0-5,0 Gew.-%, bevorzugter 0,01-2,5 Gew.-%, noch bevorzugter 0,1-1,0 Gew.-%, insbesondere 0,3-0,5 Gew.-%. In einer bevorzugten Ausführungsform der Erfindung enthält die Zusammensetzung keine Polyacrylsäure.

In einer bevorzugten Ausführungsform der Erfindung enthält die Polymermatrix neben dem Gelgerüstbildner als Verdickungsmittel ein Cellulose-Derivat, es ist aber auch möglich, dass kein Cellulose-Derivat vorhanden ist.

Als bevorzugte Cellulose-Derivate können genannt werden Methylcellulose, Ethylcellulose, Propylcellulose, Hydroxyethylcellulose, Hydroxypropylcellulose, Carboxymethylcellulose, Hydroxypropylmethylcellulose, Celluloseacetatbutyrat, Celluloseacetatpropionat, Hydroxypropylmethylcelluloseacetatsuccinat und Methylhydroxypropylcellulosephthalat oder Mischungen davon. Ethylcellulose, Hydroxypropylcellulose und Hydroxypropylmethylcellulose sind besonders bevorzugt, insbesondere Ethylcellulose und Hydroxypropylcellulose, wobei Hydroxypropylcellulose am bevorzugtesten ist.

Erfindungsgemäß bevorzugte Cellulose-Derivate sind Verbindungen, welche Untereinheiten der allgemeinen Formel (VI) 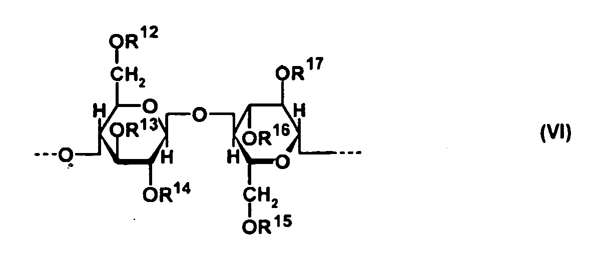 aufweisen,
worin R¹², R¹³, R¹⁴, R¹⁵, R¹⁶ und R¹⁷ jeweils unabhängig voneinander Wasserstoff oder ein linearer oder verzweigter C₁-C₆₁ Alkylrest sind, dessen Kohlenstoffkette bis zu 20mal mit Sauerstoffatomen unterbrochen sein kann und der gegebenenfalls substituiert sein kann mit 1 oder 2 Hydroxylgruppen, Carboxylgruppen oder Acyloxygruppen, wobei sich die Acyloxygruppen gegebenenfalls von einer C₁-C₇ Carbonsäure oder C₁-C₇ Dicarbonsäure ableiten. Bevorzugt sind R¹², R¹³, R¹⁴, R¹⁵, R¹⁶ und R¹⁷ jeweils unabhängig voneinander Wasserstoff oder ein linearer oder verzweigter C₁-C₁₉ Alkylrest, dessen Kohlenstoffkette bis zu 6mal mit Sauerstoffatomen unterbrochen sein kann und der gegebenenfalls substituiert sein kann mit 1 oder 2 Hydroxylgruppen.

Die einzelnen Untereinheiten der allgemeinen Formel (VI) können innerhalb des Cellulose-Derivats verschieden substituiert sein. So ist es beispielsweise möglich, dass bei einer ersten Untereinheit R¹², R¹³, R¹⁴, R¹⁵ und R¹⁶ -CH₂CH₃ sind und R¹⁷ -H ist, während bei einer anderen Untereinheit R¹³, R¹⁴, R¹⁵, R¹⁶ und R¹⁷ -CH₂CH₃ sind und R¹² -H ist.

Die erfindungsgemäß bevorzugten Cellulose-Derivate weisen bevorzugt einen hohen Substitutionsgrad bezogen auf die Hydroxylgruppen der Cellulose auf. Der Substitutionsgrad ausgedrückt als molare Substitution (MS) ist bevorzugt von 2,0 bis 6,0, stärker bevorzugt 3,0 bis 6,0, stärker bevorzugt 3,0 bis 5,0, insbesondere 3,4 bis 4,4.

Besonders bevorzugte Substituenten R¹², R¹³, R¹⁴, R¹⁵, R¹⁶ und R¹⁷ sind: -H, -CH₃, -CH₂CH₃, -CH₂CH₂CH₃, -CH(CH₃)₂, -CH₂CH₂CH₂CH₃, -CH₂CH₂OH, -CH₂CH(CH₃)OH, -[CH₂CH(CH₃)O]ₓH, -CH₂CH(CH₃)OCH₃ und -[CH₂CH(CH₃)O]ₓCH₃,
worin x jeweils eine Zahl von 2 bis 20, bevorzugt von 2 bis 6, insbesondere 2, 3 oder 4 sein kann.

Erfindungsgemäß bevorzugt handelt es sich bei der Untereinheit der allgemeinen Formel (VI) um Untereinheiten der Hydroxypropylcellulose, d.h. die Reste R¹², R¹³, R¹⁴, R¹⁵, R¹⁶ und R¹⁷ sind vorzugsweise unabhängig voneinander -H, - CH₂CH(CH₃)OH oder -[CH₂CH(CH₃)O]ₓH, worin x jeweils eine Zahl von 2 bis 20, bevorzugt von 2 bis 6 sein kann. Hydroxypropylcellulose kann durch Umsetzung von Alkalimetallsalzen der Cellulose mit Propylenoxid und anschließende Neutralisation der Alkoxygruppen erhalten werden. Reagiert eine der Hydroxylgruppen der Cellulose bei dieser Umsetzung nicht mit Propylenoxid, so ist R¹², R¹³, R¹⁴, R¹⁵, R¹⁶ bzw. R¹⁷ -H. Reagiert eine der Hydroxylgruppen der Cellulose bei dieser Umsetzung mit einem Äquivalent Propylenoxid, so ist R¹², R¹³, R¹⁴, R¹⁵, R¹⁶ bzw. R¹⁷ - CH₂CH(CH₃)OH. Reagiert eine der Hydroxylgruppen der Cellulose bei dieser Umsetzung mit einem Äquivalent Propylenoxid und reagiert das daraus hervorgegangene Alkoxid mit weiteren Äquivalenten Propylenoxid, so ist R¹², R¹³, R¹⁴, R¹⁵, R¹⁶ bzw. R¹⁷ -[CH₂CH(CH₃)O]ₓH. Theoretisch kann dabei der Index x eine beliebige Zahl sein, je nachdem, wie viele Äquivalente Propylenoxid oligomerisieren. Bevorzugt ist der Index x eine Zahl von 2 bis 20, bevorzugter von 2 bis 6, insbesondere 2, 3 oder 4.

Besonders bevorzugt haben R¹², R¹³, R¹⁴, R¹⁵, R¹⁶ und R¹⁷ jeweils unabhängig voneinander eine der folgenden Bedeutungen: -H, -CH₂CH(CH₃)OH, -[CH₂CH(CH₃)O]₂H oder -[CH₂CH(CH₃)O]₃H.

Besonders bevorzugt ist erfindungsgemäß eine Methylcellulose, eine Hydroxypropylmethyl-cellulose, eine Ethylcellulose und insbesondere eine Hydroxypropylcellulose, jeweils mit einer molaren Substitution (MS) von 3,0 bis 6,0, stärker bevorzugt 3,0 bis 5,0, insbesondere von 3,4 bis 4,4.

Bevorzugte Cellulose-Derivate sind dem Fachmann bekannt. Ethylcellulose, Hydroxypropylcellulose und Hydroxypropylmethylcellulose sind beispielsweise unter den Bezeichnungen Klucel^{®}, Klucel^{®} EXF (Aqualon^{®}), Lucel^{®} HF und Tylopur^{®} MH 1000 im Handel erhältlich. Klucel^{®} HF ist erfindungsgemäß besonders bevorzugt. Bezüglich weiterer Informationen kann beispielsweise *auf* Fiedler, Lexikon der Hilfsstoffe, ECU Aulendorf 1996, 4. Auflage *und* Hunnius Studienausgabe, de Gruyter 1993, 7. Auflage, verwiesen werden. Bezüglich weiterer Informationen über Hydroxypropylcellulose vom Typ Klucel^{®} kann auf Hercules, Aqualon, Klucel® - Physical and Chemical Properties, Produktspezifikation, Hercules Incorporated 2001 verwiesen werden.

Das gewichtsmittlere Molekulargewicht M_{w} des Cellulose-Derivats liegt bevorzugt im Bereich von 50.000 bis 2.000.000 gmol⁻¹, bevorzugter im Bereich von 300.000 bis 1.500.000 gmol⁻¹, noch bevorzugter im Bereich von 750.000 bis 1.250.000 gmol⁻¹, insbesondere im Bereich von 850.000 bis 1.150.000 gmol⁻¹.

Die Brookfield Viskosität des Cellulose-Derivats in Wasser bei 25°C in einer Konzentration von 1 % beträgt bevorzugt 1.000 bis 4.000 mPas, bevorzugter 1.275 bis 3.500 mPas, insbesondere 1.500 bis 3.000 mPas. Die Messung wird mit einem Brookfield Viskosimeter, Model LVF mit 4 Spindeln und 4 Geschwindigkeiten durchgeführt, mit dem der Bereich von 0 bis 100.000 mPas abgedeckt werden kann.

Bevorzugt beträgt der Gewichtsanteil des Cellulose-Derivats an der pharmazeutischen Zusammensetzung 0-5,0 Gew.-%, bevorzugter 0,01-2,5 Gew.-%, noch bevorzugter 0,1-1,5 Gew.-%, insbesondere 0,3-1,0 Gew.-%.

Erfindungsgemäß bevorzugt umfasst die Polymermatrix neben dem Acrylpolymer als Gelgerüstbildner alternativ als Verdickungsmittel entweder Polyacrylsäure oder ein Cellulose-Derivat. Die Kombination eines Acrylpolymers als Gelgerüstbildner mit Hydroxypropylcellulose als Verdickungsmittel ist besonders bevorzugt.

Die erfindungsgemäß verwendeten pharmazeutischen Zusammensetzungen können Fettsäuren mit mehr als 12 Kohlenstoffatomen oder deren Ester bzw. die von diesen Fettsäuren abgeleiteten Fettalkohole oder primären Amine und/oder C₁-C₁₈-Alkylether von Mono-, Di-, Tri- oder Tetraethylenglykol, insbesondere Diethylenglykolmonoethylether, und/oder Terpene enthalten. Diese Verbindungen sind jedoch keine notwendigen Bestandteile der erfindungsgemäßen pharmazeutischen Zusammensetzungen, und die erfindungsgemäßen pharmazeutischen Zusammensetzungen enthalten bevorzugt keine dieser Verbindungen.

In einer besonders bevorzugten Ausführungsform enthält die pharmazeutische Zusammensetzung die folgenden Bestandteile in folgenden Gewichtsanteilen:

**Tabelle 1:**

| **Bestandteil** | **Anteil [Gew.-%]** |
|---|---|
| eF-MENT | 0,01-5,0 |
| Acrylat/C10-30 alkylacrylat Crosspolymer | 0,1-1,5 |
| Polyacrylsäure | 0-1,0 |
| Cellulose-Derivat | 0-2,0 |
| Propylencarbonat | 5,0-20,0 |
| Glycerin 86% | 0,01-5,0 |
| Cyclomethicon | 0,01-5,0 |
| Isopropylmyristat | 0,01-5,0 |
| gereinigtes Wasser | 20,0-50,0 |
| Ethanol | 30,0-60,0 |
| Tris-(hydroxymethyl)-aminomethan | ad pH 5-7 |

Die offenbarten pharmazeutischen Zusammensetzungen eignen sich als Medikament. Die zu behandelnde Indikation bestimmt den zu verabreichenden Wirkstoff. Offenbarte Formulierungen, die Steroide enthalten, können beispielsweise zur Vorbeugung und/oder Behandlung verschiedener Steroid-Mangelerscheinungen verwendet werden. Als beispielhafte Anwendungen können die Androgenersatztherapie, Kontrazeption, primärer und sekundärer Hypogonadismus, testikuläre Fehlfunktion, Haarausfall, Alterung, Verlust and Knochensubstanz, Muskelschwund, erektile Disfunktion, benigne Prostatahypertrophie und Prostatakrebs genannt werden. Die Verwendung in der Kontrazeption ist Gegenstand der vorliegenden Erfindung. Die Anmeldung offenbart weiterhin die Eignung von Zusammensetzungen, welche MENT, eF-MENT, MENTAc oder eF-MENTAc enthalten, zur Therapie bzw. Vorbeugung von primärem und sekundärem Hypogonadismus. Bezüglich der Pharmakologie, Biologie und der klinischen Anwendungen von Androgenen kann auf Mutschler Arzneimittelwirkungen-Lehrbuch der Pharmakologie und Toxikologie, 2001*,* S. Bhasin et al., Pharmacology, Biology, and Clinical Applications of Andro-gens: Current Status and Future Prospects, John Wiley & Sons, 1st ed., 1996*,* Ch. Chawnshang, Androgens and Androgen Receptor: Mechanisms, Functions, and Clinical Applications, Kluwer Academic Publishers, 2002 und M. Carruthers, Androgen Deficiency in the Aging Male, CRC Press-Parthenon Publishers, 1st ed., 2002 verwiesen werden.

Die erfindungsgemäß verwendeten pharmazeutischen Zusammensetzungen sind zur systemischen Verabreichung des Wirkstoffs durch lokales Auftragen auf der Haut konfektioniert. Die Zusammensetzungen können entweder mit der Hand oder einem geeigneten Hilfsmittel, wie z.B. einem Spatel auf der Haut durch Verstreichen aufgetragen werden, es ist aber auch möglich, die Zusammensetzungen in Form von Sprays auf die Haut aufzutragen.

Die Erfindung betrifft auch die Verwendung eines Kohlensäurediesters zur Verbesserung der transdermalen Permeation eines Wirkstoffs in einer pharmazeutischen Zusammensetzung in Form eines Hydrogels, wobei die Zusammensetzung weiterhin bevorzugt einen C₂-C₄ Alkylalkohol, bevorzugt Ethanol, und eine Polymermatrix umfasst. Bezüglich der bevorzugten Bestandteile (d.h. bezüglich der Wirkstoffe, der C₂-C₄ Alkylalkohole, der Kohlensäurediester, der Polymermatrix, der Hilfsstoffe, der Puffersubstanzen, etc.) und bezüglich der bevorzugten Mengenverhältnisse dieser Bestandteile in der pharmazeutischen Zusammensetzung kann auf die vorstehenden Ausführungen verwiesen werden. Damit betrifft die Erfindung die Verwendung eines Kohlensäurediesters, bevorzugt einer Verbindung der allgemeinen Formel (I), bevorzugter einer Verbindung der allgemeinen Formel (II) und insbesondere die Verwendung von Propylencarbonat zur Verbesserung der transdermalen Permeation eines oder mehrerer Wirkstoffe in einer pharmazeutischen Zusammensetzung in Form eines Hydrogels, wobei das Hydrogel bevorzugt eine Polymermatrix und einen C₂-C₄ Alkylalkohol umfasst und bevorzugt wie vorstehend definiert ist.

Erfindungsgemäß wurde ebenfalls überraschend gefunden, dass eine pharmazeutische Zusammensetzung in Form eines Hydrogels, welche ein Acrylpolymer in Kombination mit einem Cellulose-Derivat umfasst, das Problem nicht mehr aufweist, dass das Gelgerüst zerstört wird und das Gel zerläuft, wenn das Gel auf schweißnasse Haut aufgetragen wird (schweißfeste Zusammensetzung). Zur Erzielung dieses Effekts ist die Anwesenheit eines Kohlensäurediesters in der Formulierung nicht erforderlich.

Erfindungsgemäß umfasst eine derartige pharmazeutische Zusammensetzung (schweißfeste Zusammensetzung) ein Acrylpolymer in Kombination mit einem Cellulose-Derivat.

Als Cellulose-Derivat enthält eine derartige pharmazeutische Zusammensetzung (schweißfeste Zusammensetzung) bevorzugt ein Cellulose-Derivat, wie es vorstehend definiert wurde, insbesondere eine Verbindung ausgewählt aus der Gruppe bestehend aus Ethylcellulose, Hydroxypropylcellulose und Hydroxypropylmethylcellulose. Ethylcellulose und Hydroxypropylcellulose sind besonders bevorzugt, wobei Hydroxypropylcellulose bevorzugter ist. Die molare Substitution des Cellulose-derivats ist bevorzugt 2 bis 6, stärker bevorzugt 3 bis 6, insbesondere 3 bis 5, z.B. 3,4 bis 4,4.

Als Acrylpolymer enthält eine derartige pharmazeutische Zusammensetzung (schweißfeste Zusammensetzung) bevorzugt ein Acrylpolymer, wie es vorstehend definiert wurde, insbesondere ein Homopolymer oder Copolymer, welches sich von Acrylsäure, Methacrylsäure, Acrylsäure-C₁-C₃₀-alkylester und/oder Methacrylsäure-C₁-C₃₀-alkylester ableitet. Besonders bevorzugt ist ein Copolymer, welches sich von einer Mischung aus Acrylsäure und Acrylsäure-C₁₀-C₃₀-alkylestern ableitet. In einer besonders bevorzugten Ausführungsform handelt es sich um ein Acrylat/C10-30 Alkylacrylat Crosspolymer.

Das relative Gewichtsverhältnis zwischen dem Acrylpolymer und dem Cellulose-Derivat in der pharmazeutischen Zusammensetzung (schweißfesten Zusammensetzung) beträgt bevorzugt 0,1-10,0, bevorzugter 0,2-5,0, noch bevorzugter 0,5-2,0, insbesondere 0,75-1,75.

Der Gesamtanteil des Acrylpolymers und des Cellulose-Derivats an der pharmazeutischen Zusammensetzung (schweißfeste Zusammensetzung) beträgt bevorzugt 0,01-20,0 Gew.-%, bevorzugter 0,1-10,0 Gew.-%, noch bevorzugter 0,3-5,0 Gew.-%, insbesondere 1,0-2,0 Gew.-%.

In einer bevorzugten Ausführungsform enthält die erfindungsgemäß verwendete pharmazeutische Zusammensetzung (schweißfeste Zusammensetzung) 0,5-1,0 Gew.-% eines Acrylat/C10-30 Alkylacrylat Crosspolymers in Kombination mit 0,2-0,8 Gew.-% Hydroxypropyl-cellulose.

Bevorzugt handelt es sich bei der erfindungsgemäß verwendeten pharmazeutischen Zusammensetzung (schweißfesten Zusammensetzung) um ein Hydrogel. Der Wasser-gehalt des Hydrogels beträgt bevorzugt 5,0-90,0 Gew.-%, bevorzugter 10,0-70,0 Gew.-%, noch bevorzugter 20,0-50,0 Gew.-%, insbesondere 25,0-40,0 Gew.-%.

Bevorzugt enthält die erfindungsgemäß verwendete pharmazeutische Zusammensetzung (schweißfeste Zusammensetzung) einen C₂-C₄ Alkylalkohol, wobei Ethanol besonders bevorzugt ist. Für diesen Fall enthält die pharmazeutische Zusammensetzung (schweißfeste Zusam-mensetzung) bevorzugt 29,0-73,0 Gew.-% Ethanol und 5,0-50,0 Gew.-% Wasser, bevorzugter 34,0-68,0 Gew.-% Ethanol und 10,0-45,0 Gew.-% Wasser, noch bevor-zugter 39,0-63,0 Gew.-% Ethanol und 15,0-40,0 Gew.-% Wasser, besonders bevor-zugt 44,0-58,0 Gew.-% Ethanol und 20,0-35,0 Gew.-% Wasser und insbesondere 54,8-57,5 Gew.-% Ethanol und 27,2-30,8 Gew.-% Wasser.

Die erfindungsgemäß verwendete pharmazeutische Zusammensetzung (schweißfeste Zusammensetzung) enthält neben dem Acrylpolymer und dem Cellulose-Derivat gegebenenfalls mindestens einen Wirkstoff und weitere Inhaltsstoffe, wie z.B. Hautpflegemittel, Hilfsstoffe, Lösungsmittel, Permeationsvermittler, etc. Der Wirkstoff, die gegebenenfalls vorhandenen weiteren Inhaltsstoffe und ihre bevorzugten prozentualen Anteile an der Zusammensetzung sind wie vorstehend definiert.

Damit umfasst die erfindungsgemäß verwendete pharmazeutische Zusammensetzung (schweißfeste Zusammensetzung) in Form eines Hydrogels bevorzugt einen Wirkstoff und/oder einen Kohlensäurediester und/oder einen C₂-C₄ Alkylalkohol und/oder eine Polymermatrix wie vorstehend definiert. Sowohl die bevorzugten prozentualen Gewichtsanteile an der Zusammensetzung und relativen Gewichtsverhältnisse der einzelnen Bestandteile untereinander als auch die bevorzugten Wirkstoffe, bevorzugten Kohlensäurediester und bevorzugten C₂-C₄ Alkylalkohole sind wie vorstehend definiert.

Die erfindungsgemäß verwendete pharmazeutische Zusammensetzung (schweißfeste Zusammensetzung) kann anstelle des Kohlensäurediesters oder in Ergänzung zum Kohlensäurediester einen Permeationsvermittler enthalten, der ausgewählt ist aus der Gruppe bestehend aus:
(i) aliphatischen Fettsäureestern, welche 10-30 Kohlenstoffatome enthalten und gegebenenfalls substituiert sind mit 1-2 Hydroxylgruppen, Carboxylgruppen oder C₁-C₄ Acyloxygruppen;
(ii) aliphatischen Fettsäurealkoholen, welche 10-30 Kohlenstoffatome enthalten und gegebenenfalls substituiert sind mit 1-2 Hydroxylgruppen, Carboxylgruppen oder C₁-C₄ Acyloxygruppen; oder
(iii) einer Verbindung der allgemeinen Formel (VII)

   HO-(CH₂CH₂-O)ₙ-R¹⁸ (VII)
worin R¹⁸ C₁-C₁₂-Alkyl, C₁-C₁₂-Alkenyl, C₁-C₁₂-Alkanoyl oder C₁-C₁₂-Alkenoyl und der Index n eine Zahl von 1 bis 10 ist.

Bevorzugt enthält die erfindungsgemäß verwendete pharmazeutische Zusammensetzung (schweißfeste Zusammensetzung) eine Verbindung der allgemeinen Formel (VII), worin R¹⁸ C₁-C₄-Alkyl und der Index n eine Zahl von 1 bis 3 ist.

Besonders bevorzugte Verbindungen der allgemeinen Formel (VII) sind: HOCH₂CH₂O-CH₃, HO-CH₂CH₂O-CH₂CH₃, HO-CH₂CH₂O-CH₂CH₂CH₃, HO-CH₂CH₂O-CH₂CH₂CH₂CH₃, HO-(CH₂CH₂O)₂-CH₃, HO-(CH₂CH₂O)₂-CH₂CH₃, HO-(CH₂CH₂O)₂-CH₂CH₂CH₃, HO-(CH₂CH₂O)₂-CH₂CH₂CH₂CH₃, HO-(CH₂CH₂O)₃-CH₃, HO-(CH₂CH₂O)₃-CH₂CH₃, HO-(CH₂CH₂O)₃-CH₂CH₂CH₃, HO-(CH₂CH₂O)₃-CH₂CH₂CH₂CH₃, HO-(CH₂CH₂O)₄-CH₃, HO-(CH₂CH₂O)₄-CH₂CH₃, HO-(CH₂CH₂O)₄-CH₂CH₂CH₃ und HO-(CH₂CH₂O)₄-CH₂CH₂CH₂CH₃.

Für die erfindungsgemäß verwendete pharmazeutische Zusammensetzung (schweißfeste Zusammensetzung) ist insbesondere eine Ausführungsform bevorzugt, welche eine Verbindung der allgemeinen Formel (VII) enthält, worin R¹⁸ Ethyl und der Index n = 2 ist, d.h. Diethylenglykolmonoethylether.

Die folgenden Beispiele dienen zur weiteren Erläuterung der Erfindung.

### Beispiel 1:

Zwei erfindungsgemäß verwendete pharmazeutische Zusammensetzungen (Typ A und B) wurden auf übliche Art und Weise hergestellt. Die Bestandteile der Zusammensetzungen sind in Tabelle 2 dargestellt:

**Tabelle 2:**

| **Bestandteil** | **Menge [Gew.-%]** | |
|---|---|---|
| | **Typ A** | **Typ B** |
| Propylencarbonat | 10,0 | 10,0 |
| Ethanol | 55,0 | 55,0 |
| gereinigtes Wasser | ca. 30 | ca. 30 |
| eF-MENT | 0,8 | 0,8 |
| Acrylat/C10-30 Alkylacrylat Cross-polymer (Pemulen TR-1) | 0,8 | 0,8 |
| Polyacrylsäure | - | 0,4 |
| Hydroxypropylcellulose (Klucel HF) | 0,35 | - |
| Glycerin 86% | 1,0 | 1,0 |
| Cyclomethicon | 1,5 | 1,5 |
| Isopropylmyristat | 0,5 | 0,5 |
| Tris-(hydroxymethyl)aminomethan | ad pH 5,8 | ad pH 5,8 |

### Beispiel 2 (Best Mode):

Eine pharmazeutische Zusammensetzung (schweißfeste Zusammensetzung, Typ C) in Form eines Hydrogels, welche ein Acrylpolymer in Kombination mit einem Cellulose-Derivat umfasst, wurde ohne Wirkstoff hergestellt. Die Bestandteile der Zusammensetzungen sind in Tabelle 3 dargestellt:

**Tabelle 3:**

| **Bestandteil** | **Menge [Gew.-%]** **Typ C** |
|---|---|
| Propylencarbonat | 10,0 |
| Ethanol | 55,0 |
| gereinigtes Wasser | ca. 30 |
| Acrylat/C10-30 Alkylacrylat Cross-polymer (Pemulen TR-1) | 0,8 |
| Hydroxypropylcellulose (Klucel HF) | 0,35 |
| Glycerin 86% | 1,0 |
| Cyclomethicon | 1,5 |
| Isopropylmyristat | 0,5 |
| Tris-(hydroxymethyl)aminomethan (Tromethamin) | ad pH 5,8 |

Es wurde das folgende Herstellungsverfahren durchgeführt:

Pemulen TR-1 und HPC (Klucel HF) wurden in Ethanol 96% aufgequollen. Es wurde Propylencarbonat zugegeben und unter Rühren gemischt. Anschließend wurde Isopropylmyristat zugegeben, und es wurde erneut gemischt. Der Gelbildner und der Quellstoff begannen im Lösungsmittelgemisch aufzuquellen. Das Gesamtgemisch wurde über einen Trichter in ein Mischer/Homogenisator-System (Becomix RW 2,5) eingebracht (in den Becomix RW 2,5 eingesogen) und mit 20 rpm kurz gerührt. Danach wurde für 1 Minute mit 2000-3500 rpm homogenisiert (z.B. mit einem Rotor-Stator-Homogenisator). Man erhielt ein homogenes, klares Gel frei von Agglomeraten, welches noch nicht vollständig aufgequollen war. Glycerin 86%, und die Gesamtmenge des gereinigten Wassers wurde als schlierenfreies Gemisch in mehreren Teilschritten zugegeben (eingesogen), und das Gel quoll nochmals deutlich nach. Es wurde solange gerührt (ca. 5 Minuten), bis eine deutliche Auflärung des Gels sichtbar wurde. Mit jeder Wasser/Glycerin-Zugabe bildete sich das Gelgerüst besser auf und wurde zugleich auch immer klarer. Die Drehzahl betrug 50 rpm. Anschließend wurde Cyclomethicon unter Rühren in das Gemisch eingearbeitet. Damit eine gleichmäßige Verteilung der Wasser/Glycerin-Mischung sowie des Cyclomethicons gewährleistet ist, wurde am Ende der Einarbeitung nochmals 1 Minute mit 4000 rpm homogenisiert. Anschließend wurde unter Rühren bei 50 rpm in 3 Teilschritten eine 10%ige wässrige Tromethamin-Lösung zur Neutralisation zugegeben. Das Gel klarte dabei nochmals deutlich auf, und die Gelstruktur baute sich weiter auf. Am Ende der Einarbeitung wurde nochmals 2 Minuten mit 2700 rpm homogenisiert.

### Beispiel 3:

In einer Versuchsreihe wurden 3 verschiedene pharmazeutische Zusammensetzungen in Form von Hydrogelen im Hinblick auf ihre Hautverträglichkeit untersucht. Die Formulierungen waren wie folgt:
Vergleichsformulierung 1 (VB1) (Beispiel entsprechend der Offenbarung der EP-A 817 621):
   Ethanol 70.2%, Ethylhexyl Ethylhexanoat 20%, Cetearyloctanoat 1%; Hydroxypropylcellulose 1.5% ; Wasser 3.6%
Vergleichsformulierung 2 (VB2); Androgel (Marktprodukt):
   68,9% Ethanol; Isopropylmyristat, Polyacrylsäure, Wasser, NaOH

Erfindungsgemäße Formulierung 3 (EB3), hergestellt entsprechend dem Verfahren des Beispiels 2:
Ethanol 45%, Propylencarbonat 10%, PEG 400 2.0%, Acrylpolymer 0.6%, Glycerin 4.0%, Wasser 37.25%, Diisopropylamin q.s.

Es wurde die lokale Verträglichkeit am Kaninchen nach einmal täglicher dermaler Applikation von 0,25 g pro Applikationsstelle über 2 Wochen (insgesamt 14 Applikationen) auf der intakten Haut untersucht. Die Untersuchung erfolgte nach der EMEA Guideline (CPMP/SWP/2145/00). Die Untersuchung erfolgte an 6 männlichen Tieren (Weiße Neuseeländer/konv.).

Als Negativkontrolle diente Leitungswasser, als Positivkontrolle Testogel / Androgel. Die Expositionszeit betrug 4 h. Behandlungsrückstände wurden mit lauwarmem Wasser entfernt. Die Ablesung und Bewertung der Reaktion erfolgte jeweils am Ende der Expositionszeit vor dem Entfernen des Substanzrestes. Die Tiere wurden am Tag nach der letzten Behandlung (Tag 15) getötet und seziert. Die Probennahme sowie die histologische Aufarbeitung erfolgten nach den Angaben der SOP TX ME Nr. 382.4.

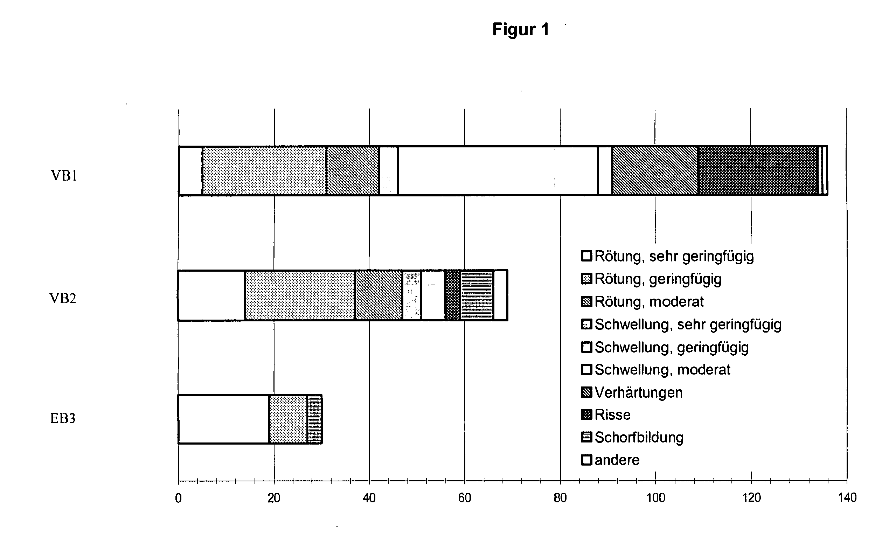

Die Ergebnisse sind in Figur 1 gezeigt, wobei die X-Achse die Anzahl der Indikationen angibt und auf der Y-Achse die Formulierungen aufgeführt sind.

Wie aus den Vergleichsversuchen hervorgeht, ist die erfindungsgemäße Zusammensetzung den übrigen Zusammensetzungen des Standes der Technik überlegen. Wie aus Vergleichsformulierungen 1 und 2 (VB1 und VB2) hervorgeht, zeigen Gele, welche einen hohen Ethanolgehalt aufweisen, deutliche Nebenwirkungen. Dabei fällt auf, dass die Nebenwirkungen nicht ausschließlich durch das Ethanol hervorgerufen werden, sondern offenbar auch von der Art des verwendeten Permeationsvermittlers abhängig sind. Bei einem Ethanolgehalt von 70 Gew.-% zeigt das vermarktete Produkt (Androgel) deutlich weniger Nebenwirkungen als Vergleichsformulierung 1 (VB1), bei welchem Ethylhexyl ethylhexanoat als Permeationsvermittler verwendet wurde (vgl. Produkt gemäß EP-A 817 621).

### Beispiel 4:

Rheologische Charakterisierung der Elektrolyttoleranz von Gelsystemen auf Basis von Polyacrylsäure (z.B. Carbopol) versus Gelsystemen auf Basis einer Kombination aus Hydroxypropylcellulose und Pemulen TR-1 (schweißfeste Zusammensetzung).

Die Versuchsanordnung simuliert die "Schweißfestigkeit" der beschriebenen Gelsysteme auf der Haut. Dazu wurden ausgewählte Elektrolyt-Zubereitungen zu der Zusammensetzung gegeben und die rheologischen Parameter bestimmt. Die Elektrolyttoleranz wird durch Vergleich der unbehandelten mit der elektrolytbelasteten Probe charakterisiert.

Die Zubereitungen wurden mit 0,01 % NaCl in kristalliner Form versetzt und das Salz wurde unter moderatem Rühren eingearbeitet. Anschließend erfolgte sofort die rheologische Messung. Die Messapparatur bestand aus einem Rotationsviskosimeter RC 20 der Fa. Europhysics mit Peltierthermostat. Die Messtemperatur betrug 25°C, die Vorgabe der Schubspannung (τ) betrug 150 Pascal. Es wurde der Messkegel C50-1 verwendet.
1. Androgel auf Basis von Polyacrylsäure (Carbopol) (Marktprodukt Androgel)
2. erfindungsgemäßes Vehikel auf Basis von Hydroxypropylcellulose (Klucel HF) / Pemulen TR-1, Hydrogel gemäß Beispiel 2

Die Fließgrenze des Androgels fiel durch die Elektrolytbelastung auf ca. 17% des Ausgangswerts ab. Die Fließgrenze der erfindungsgemäßen Variante auf Basis von Hydroxypropylcellulose / Pemulen TR-1 reduzierte sich hingegen lediglich auf ca. 89 % Ausgangswerts.

**Tabelle 4:**

| **Charge** | **Fließgrenze (Pa)** | **Restfließgrenze ( in Prozent des Ausgangswerts)** |
|---|---|---|
| Androgel ohne Elektrolyt | 15,3 | 16,7 |
| Androgel mit NaCl 0,01 % | 2,5 | |
| erfindungsgemäßes Vehikel ohne Elektrolyt | 22,9 | 89,5 |
| erfindungsgemäßes Vehikel mit NaCl 0,01 % | 20,3 | |

Die Ergebnisse zeigen für das erfindungsgemäße System auf Basis von Hydroxypropylcellulose / Pemulen TR-1 eine deutlich höhere Elektrolyttoleranz. Dieses Verhalten korreliert mit den Anwendungserfahrungen in der Praxis. Die erfindungsgemäße Zubereitung lässt sich ohne Zerlaufen und Abtropfen von der Haut applizieren. Dagegen kommt es zum Zerlaufen und Abtropfen des Marktprodukts Androgel, was durch die starke Reduktion der Fließgrenze bedingt ist.

### Beispiel 5:

Das Permeationsverhalten in Bezug auf den Wirkstoff eF-MENT einer erfindungsgemäßen Zusammensetzung, welche 55 Gew.-% Ethanol und 10 Gew.-% Propylencarbonat enthielt und die gemäß Beispiel 2 vorstehend hergestellt worden war, wurde mit dem Permeationsverhalten aus einer Zusammensetzung verglichen, welche in etwa der Zusammensetzung des Marktprodukts Androgel entsprach.

Die Experimente wurde mit Hilfe einer Franz-Diffusionszelle durchgeführt (vgl. T.J. Franz, Invest. Dermato/. 1975, 64, 191). Dieses Modell besteht aus einer Diffusionszelle, welche in ein Donor-Kompartiment und ein Akzeptor-Kompatiment unterteilt werden kann. In diesem System fungiert die Haut als Barriere zwischen diesen Kompartimenten. Dabei wird die Haut derart zwischen die Kompartimente eingebracht, dass die dermale Seite durch die Lösung des Akzeptor-Kompartiments umspült wird. Das Akzeptor-Kompartiment der Diffusionszelle ist mit einer HPLC-Anlage verbunden, wodurch eine automatische Analyse von Aliquots der Lösung möglich ist.

Die konkrete Zusammensetzung des untersuchten erfindungsgemäßen Hydrogels ist in Tabelle 5 dargestellt:

**Tabelle 5:**

| **Bestandteil** | **[Gew.-%]** |
|---|---|
| Propylencarbonat | 10,0 |
| Ethanol | 55,0 |
| gereinigtes Wasser | ∼29,0 |
| eF-MENT | 0,8 |
| Acrylat/C10-30 Alkylacrylat Cross-polymer (Pemulen TR-1) | 0,8 |
| Methylcellulose (Tylopur MH 1000, erhältlich von der Firma Clariant) | 0,5 |
| Glycerin 86% | 1,0 |
| Cyclomethicon | 1,5 |
| Isopropylmyristat | 0,5 |
| Tris-(hydroxymethyl)aminomethan | ad pH 5,8 |

Es wurde die Haut von haarlosen Nacktmäusen (HsdCpb NMRI-nu/nu, Harlan Bioservice, Walsrode) untersucht.

Die Lösung im Akzeptor-Kompartiment hatte folgende Zusammensetzung:
Kaliumchlorid 0.4 g
Kaliumdihydrogenphosphat 0.06 g
Natriumchlorid 7.27 g
Natriumhydrogenphosphat Dihydrat 0.06 g
HEPES 5.96 g
Gentamicinsulfat 0.05 g
γ-Cyclodextrin 5.0 g
Aqua purificata ad 1000 g

Bei den Experimenten wurden Aliquots von 250µl der Probe auf die Oberfläche der epidermalen Seite der Haut (45 cm²) mit Hilfe einer Spritze aufgetragen. Mit den Fingerspitzen, welche durch Handschuhe geschützt waren, wurden die Proben leicht auf der Hautoberfläche verrieben. 5 Minuten nach Auftragen des Gels wurde ein Teil der Haut in die Diffusionszelle eingebracht. Die Akzeptor-Lösung der Franz-Zelle wurde kontinuierlich homogenisiert. Die zur Verfügung stehende Permeationsfläche betrug 1,5 cm². Nach festgelegten Zeitintervallen (3, 6, 9, 12, 15 und 18 h) wurden Aliquots der Akzeptor-Lösung entnommen, in die HPLC-Anlage injiziert und automatisch analysiert. Der Gehalt an eF-MENT in den Proben wurde mit Hilfe von Standardlösungen quantifiziert, wobei die Standardlösungen parallel und unter den gleichen HPLC-Bedingungen vermessen wurden.

HPLC:
Säule: Vertex mit Vorsäule, Nucleosil - 100 C18, 5 µm, 125 x 3 mm
Mobile Phase: Acetonitril / Wasser (39/61)
Flussrate: 0,700 ml/min
Temperatur: 40°C
Injektionsvolumen: 200 µl
Detektionswellenlänge: 244 nm
Retentionszeit: eF-MENT 4,8 min.

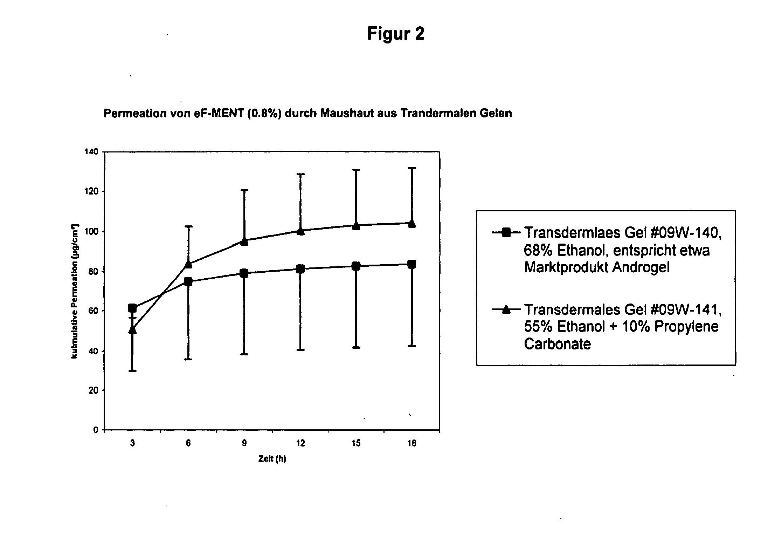

Die Ergebnisse der Untersuchungen sind in Figur 2 veranschaulicht (das transdermale Gel #09W-140 dient als Vergleichsbeispiel, das transdermale Gel #09W-141 ist das erfindungsgemäße Beispiel).

Die Experimente belegen, dass die erfindungsgemäße Zusammensetzung (#09W-141) bei verbesserter Verträglichkeit und verbesserter Applikationseigenschaft sehr gute Permeationseffizienzen hinsichtlich des transdermal zu verabreichenden Wirkstoffs aufweist.

## Patentansprüche

1. Verwendung eines Hydrogels enthaltend einen Kohlensäurediester, einen C₂-C₄ Alkylalkohol, mindestens einen Wirkstoff und eine Polymermatrix zur Herstellung eines transdermalen Kontrazeptivums.

2. Verwendung nach Anspruch 1, **dadurch gekennzeichnet, dass** der Kohlensäurediester eine Verbindung der allgemeinen Formel (I) 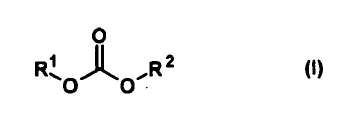 ist, worin entweder
(i) R¹ und R² unabhängig voneinander C₁-C₆-Alkyl, C₂-C₆-Alkenyl, C₂-C₆-Alkinyl,
C₄-C₆-Cycloalkyl, C₁-C₆-Heterocycloalkyl, Phenyl, C₁-C₆-Heteroaryl, Phenyl-C₁-C₄-alkyl oder C₂-C₁₀-Heteroarylalkyl sind, wobei die Alkyl-, Alkenyl- und Alkinyl-Gruppen gegebenenfalls jeweils bis zu dreimal durch Sauerstoffatome und/oder Schwefelatome unterbrochen sein können, oder
(ii) R¹ und R² unabhängig voneinander eine der oben genannten Bedeutungen haben und über eine C-C-Bindung miteinander verbunden sind.

3. Verwendung nach Anspruch 2, **dadurch gekennzeichnet, dass** der Kohlensäurediester eine Verbindung der allgemeinen Formel (II) 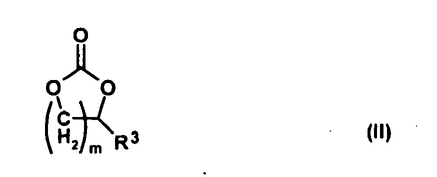 ist, worin der Index m eine Zahl von 1 bis 3 und R³ Wasserstoff oder C₁-C₄-Alkyl ist.

4. Verwendung nach Anspruch 3, **dadurch gekennzeichnet, dass** m 1 und R³ Methyl ist.

5. Verwendung nach einem der vorstehenden Ansprüche, **dadurch gekennzeichnet, dass** der C₂-C₄ Alkylalkohol Ethanol ist.

6. Verwendung nach einem der vorstehenden Ansprüche, **dadurch gekennzeichnet, dass** der Wirkstoff ein Steroid ist.

7. Verwendung nach Anspruch 6, **dadurch gekennzeichnet, dass** das Steroid ein Androgen ist.

8. Verwendung nach Anspruch 6, **dadurch gekennzeichnet, dass** das Steroid eine Verbindung der allgemeinen Formel (III) 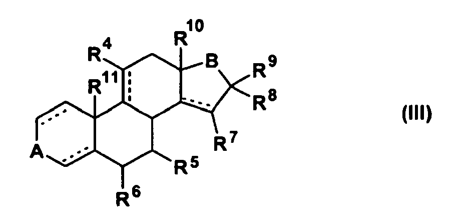 ist, worin
R⁴ Wasserstoff, Fluor, Chlor, C₁-C₃-Alkyl oder eine gegebenenfalls acetylierte Hydroxylgruppe ist,
R⁵, R⁶, R⁷, R⁸, R⁹, R¹⁰ und R¹¹ unabhängig voneinander Wasserstoff oder C₁-C₃-Alkyl sind,
die gestrichelten Linien unabhängig voneinander eine optionale Bindung sind und
A und B unabhängig voneinander eine Carbonylgruppe oder eine Gruppe 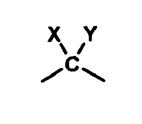 sind, worin X eine Hydroxylgruppe oder deren Ester einer Carbonsäure mit 1-8 Kohlenstoffatomen und Y Wasserstoff oder C₁-C₃-Alkyl ist.

9. Verwendung nach Anspruch 8, **dadurch gekennzeichnet, dass** R⁷, R⁸ und R⁹ Wasserstoff sind.

10. Verwendung nach Anspruch 6, **dadurch gekennzeichnet, dass** das Steroid eine Verbindung der allgemeinen Formel (IV) 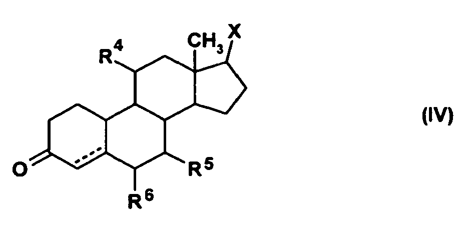 ist, worin X eine Hydroxylgruppe oder deren Ester einer Carbonsäure mit 1-4 Kohlenstoffatomen, R⁴ Wasserstoff, Fluor oder eine Hydroxylgruppe, R⁵ und R⁶ unabhängig voneinander Wasserstoff, Methyl oder Ethyl sind und die gestrichelte Linie eine optionale Bindung ist.

11. Verwendung nach Anspruch 6, **dadurch gekennzeichnet, dass** das Steroid eine Verbindung der allgemeinen Formel (V) 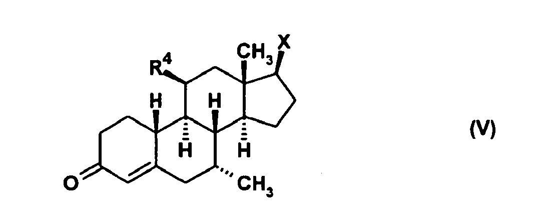 ist, worin R⁴ Fluor oder Wasserstoff und X eine Hydroxylgruppe oder deren Essigsäureester ist.

12. Verwendung nach Anspruch 11, **dadurch gekennzeichnet, dass** X eine Hydroxylgruppe ist.

13. Verwendung nach einem der Ansprüche 6 bis 12, **dadurch gekennzeichnet, dass** das Steroid in Kombination mit zumindest einem weiteren Wirkstoff vorliegt.

14. Verwendung nach einem der vorstehenden Ansprüche, **dadurch gekennzeichnet, dass** die Polymermatrix ein Acrylpolymer umfasst.

15. Verwendung nach Anspruch 14, **dadurch gekennzeichnet, dass** das Acrylpolymer ein Acrylat/C10-30 Alkylacrylat Crosspolymer umfasst.

16. Verwendung nach einem der vorstehenden Ansprüche, **dadurch gekennzeichnet, dass** die Polymermatrix ein Cellulose-Derivat umfasst.

17. Verwendung nach einem der vorstehenden Ansprüche, **dadurch gekennzeichnet, dass** sie 0,01-5,0 Gew.-% eF-MENT, 0,1-1,5 Gew.-% Acrylat/C10-30 alkylacrylat Crosspolymer, 0-1,0 Gew.-% Polyacrylsäure, 0-2,0 Gew.-% eines Cellulose-Derivats, 5,0-20,0 Gew.-% Propylencarbonat, 0,01-5,0 Gew.-% Glycerin, 0,01-5,0 Gew.-% Cyclomethicon, 0,01-5,0 Gew.-% Isopropylmyristat, 20,0-50,0 Gew.-% gereinigtes Wasser, 30,0-60,0 Gew.-% Ethanol und Tris-(hydroxymethyl)-aminomethan ad pH 5-7 Gew.-% enthält.

## Claims

1. Use of a hydrogel that contains a carbonic acid diester, a C₂-C₄ alkyl alcohol, at least one active ingredient and a polymer matrix for the production of a transdermal contraceptive.

2. Use according to claim 1, **characterized in that** the carbonic acid diester is a compound of general formula (I) 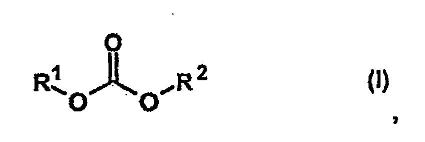 in which either
(i) R¹ and R², independently of one another, are C₁-C₆-alkyl, C₂-C₆-alkenyl, C₂-C₆-alkinyl, C₄-C₆-cycloalkyl, C₁-C₆-heterocycloalkyl, phenyl, C₁-C₆-heteroaryl, phenyl-C₁-C₄-alkyl or C₂-C₁₀-heteroarylalkyl, wherein the alkyl-, alkenyl- and alkinyl groups optionally can be interrupted in each case up to three times by oxygen atoms and/or sulfur atoms, or
(ii) R¹ and R², independently of one another, have one of the above-mentioned meanings and are connected to one another via a C-C bond.

3. Use according to claim 2, **characterized in that** the carbonic acid diester is a compound of general formula (II) 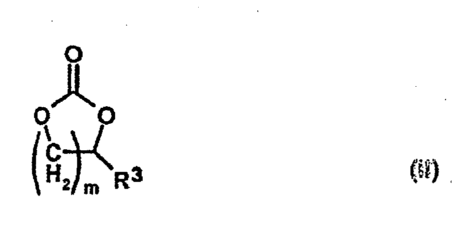 in which index m is a number from 1 to 3, and R³ is hydrogen or C₁-C₄-alkyl.

4. Use according to claim 3, **characterized in that** m is 1 and R³ is methyl.

5. Use according to any of the preceding claims, **characterized in that** the C₂-C₄ alkyl alcohol is ethanol.

6. Use according to any of the preceding claims, **characterized in that** the active ingredient is a steroid.

7. Use according to claim 6, **characterized in that** the steroid is an androgen.

8. Use according to claim 6, **characterized in that** the steroid is a compound of general formula (III) 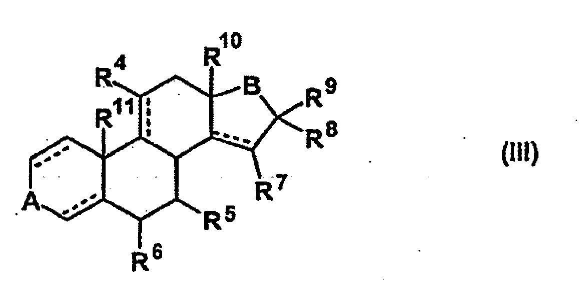 in which
R⁴ is hydrogen, fluorine, chlorine, C₁-C₃-alkyl or an optionally acetylated hydroxyl group,
R⁵, R⁶, R⁷, R⁸, R⁹, R¹⁰ and R¹¹, independently of one another, are hydrogen or C₁-C₃-alkyl,
the dotted lines, independently of one another, are an optional bond, and A and B, independently of one another, are a carbonyl group or a group 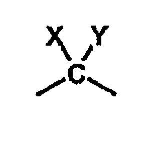 in which X is a hydroxyl group or its ester of a carbonic acid with 1-8 carbon atoms, and Y is hydrogen or C₁-C₃-alkyl.

9. Use according to claim 8, **characterized in that** R⁷, R⁸ and R⁹ are hydrogen.

10. Use according to claim 6, **characterized in that** the steroid is a compound of general formula (IV) 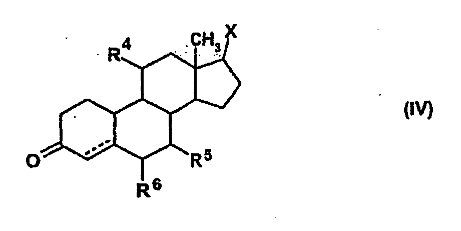 in which X is a hydroxyl group or its ester of a carbonic acid with 1-4 carbon atoms, R⁴ is hydrogen, fluorine or a hydroxyl group, R⁵ and R⁶, independently of one another, are hydrogen, methyl or ethyl, and the dotted line is an optional bond.

11. Use according to claim 6, **characterized in that** the steroid is a compound of general formula (V) 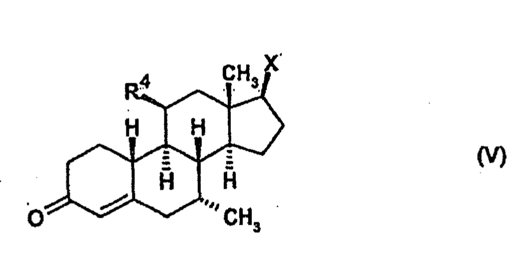 in which R⁴ is fluorine or hydrogen, and X is a hydroxyl group or its acetate.

12. Use according to claim 11, **characterized in that** X is a hydroxyl group.

13. Use according to any of claims 6 to 12,
**characterized in that** the steroid is present in combination with at least one other active ingredient.

14. Use according to any of the preceding claims, **characterized in that** the polymer matrix comprises an acrylic polymer.

15. Use according to claim 14, **characterized in that** the acrylic polymer comprises an acrylate/C10-30 alkyl acrylate crosspolymer.

16. Use according to any of the preceding claims, **characterized in that** the polymer matrix comprises a cellulose derivative.

17. Use according to any of the preceding claims, **characterized in that** it contains 0.01-5.0% by weight of eF-MENT, 0.1-1.5% by weight of acrylate/C10-30 alkyl acrylate crosspolymer, 0-1.0% by weight of polyacrylic acid, 0-2.0% by weight of a cellulose derivative, 5.0-20.0% by weight of propylene carbonate, 0.01-5.0% by weight of glycerol, 0.01-5.0% by weight of cyclomethicone, 0.01-5.0% by weight of isopropyl myristate, 20.0-50.0% by weight of purified water, 30.0-60.0% by weight of ethanol and tris-(hydroxymethyl)-aminomethane at pH 5-7.

## Revendications

1. Utilisation d'un hydrogel contenant un diester de l'acide carbonique, un alcool alkylique en C₂-C₄, au moins un principe actif, et une matrice polymère pour préparer un contraceptif transdermique.

2. Utilisation selon la revendication 1, **caractérisée en ce que** le diester de l'acide carbonique est un composé de formule générale (I) 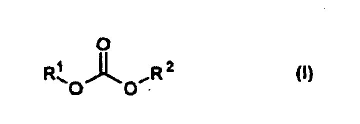 dans laquelle
(i) R¹ et R² représentent chacun indépendamment de l'autre un groupe alkyle en C₁-C₆, alcényle en C₂-C₆, alcynyle en C₂-C₆, cycloalkyle en C₄-C₆, hétérocycloalkyle en C₁-C₆, phényle, hétéroaryle en C₁-C₆, phényl (alkyle en C₁-C₄) ou hétéroarylalkyle en C₂-C₁₀, les groupes alkyle, alcényle et alcynyle pouvant chacun être éventuellement interrompus jusqu'à trois fois par des atomes d'oxygène et/ou des atomes de soufre, où
(ii) R¹ et R² ont indépendamment l'un de l'autre l'une des significations données ci-dessus, et sont reliés l'un à l'autre par une liaison C-C.

3. Utilisation selon la revendication 2, **caractérisée en ce que** le diester de l'acide carbonique est un composé de formule générale (II) 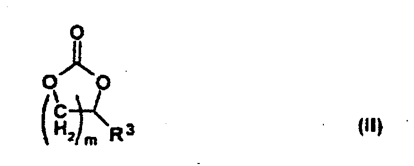 dans laquelle l'indice m est un nombre de 1 à 3, et R³ est un atome d'hydrogène ou un groupe alkyle en C₁-C₄.

4. Utilisation selon la revendication 3, **caractérisée en ce que** m vaut 1 et R³ est le groupe méthyle.

5. Utilisation selon l'une des revendications précédentes, **caractérisée en ce que** l'alcool alkylique en C₂-C₄ est l'éthanol.

6. Utilisation selon l'une des revendications précédentes, **caractérisée en ce que** la matière active est un stéroïde.

7. Utilisation selon la revendication 6, **caractérisée en ce que** le stéroïde est un androgène.

8. Utilisation selon la revendication 6, **caractérisée en ce que** le stéroïde est un composé de formule générale (III) 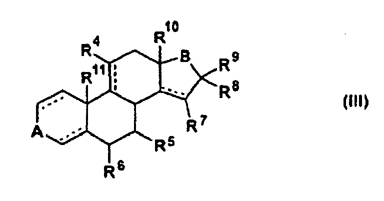 dans laquelle
R⁴ est un atome d'hydrogène, un groupe fluor, chloro, alkyle en C₁-C₃ ou un groupe hydroxyle éventuellement acétylé,
R⁵, R⁶, R⁷, R⁸, R⁹, R¹⁰ et R¹¹ représentent chacun indépendamment des autres un atome d'hydrogène ou un groupe alkyle en C₁-C₃,
les lignes en tirets représentent chacune indépendamment des autres une liaison en option, et
A et B représentent chacun indépendamment de l'autre un groupe carbonyle ou un groupe 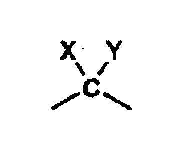 dans lequel X est un groupe hydroxyle ou son ester d'un acide carboxylique ayant 1 à 8 atomes de carbone, et Y est un atome d'hydrogène ou un groupe alkyle en C₁-C₃.

9. Utilisation selon la revendication 8, **caractérisée en ce que** R⁷, R⁸ et R⁹ sont des atomes d'hydrogène.

10. Utilisation selon la revendication 6, **caractérisée en ce que** le stéroïde est un composé de formule générale (IV) 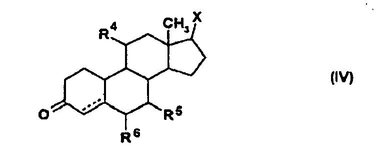 dans laquelle X est un groupe hydroxyle ou son ester d'un acide carboxylique ayant 1 à 4 atomes de carbone, R⁴ est un atome d'hydrogène ou un groupe fluoro ou hydroxyle, R⁵ et R⁶ représentent chacun indépendamment de l'autre un atome d'hydrogène, un groupe méthyle ou éthyle, et la ligne en tirets est une liaison en option.

11. Utilisation selon la revendication 6, **caractérisée en ce que** le stéroïde est un composé de formule générale (V) 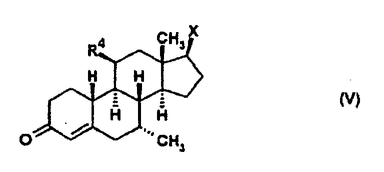 dans laquelle R⁴ est un atome de fluor ou d'hydrogène, et X est un groupe hydroxyle ou son ester de l'acide acétique.

12. Utilisation selon la revendication 11, **caractérisée en ce que** X est un groupe hydroxyle.

13. Utilisation selon l'une des revendications 6 à 12, **caractérisée en ce que** le stéroïde se présente en combinaison avec au moins un autre principe actif.

14. Utilisation selon l'une des revendications précédentes, **caractérisée en ce que** la matrice polymère comprend un polymère acrylique.

15. Utilisation selon la revendication 14, **caractérisée en ce que** le polymère acrylique comprend un polymère croisé acrylate/acrylate d'alkyle en C₁₀₋₃₀.

16. Utilisation selon l'une des revendications précédentes, **caractérisée en ce que** la matrice polymère comprend un dérivé de la cellulose.

17. Utilisation selon l'une des revendications précédentes, **caractérisée en ce qu'**elle contient 0,01-5,0 % en poids d'eF-MENT, 0,1-1,5 % en poids d'un copolymère croisé acrylate/acrylate d'alkyle en C₁₀₋₃₀, 0-1,0 % en poids de poly(acide acrylique), 0-2,0 % en poids d'un dérivé de la cellulose, 5,0-20,0 % en poids de carbonate de propylène, 0,01-5,0 % en poids de glycérol, 0,01-5,0 % en poids de cyclométhicon, 0,01-5,0 % en poids de myristate d'isopropyle, 20,0-50,0 % en poids d'eau purifiée, 30,0-60,0 % en poids d'éthanol, et du tris(hydroxyméthyl)aminométhane qsp pH 5-7 % en poids.
